(19) Europäisches Patentamt
European Patent Office
Office européen des brevets

(11) Numéro de publication: **0 304 665 B1**

(12) # FASCICULE DE BREVET EUROPEEN

(45) Date de publication de fascicule du brevet: **15.04.92**

(51) Int. Cl.$^5$: **C07D 239/50**, A61K 7/06

(21) Numéro de dépôt: **88112301.2**

(22) Date de dépôt: **29.07.88**

Le dossier contient des informations techniques présentées postérieurement au dépôt de la demande et ne figurant pas dans le présent fascicule.

(54) **Dérivés d'uréylène triamino- 2, 4, 6 pyrimidine oxyde-3, leur préparation et leur utilisation en cosmétique et dermopharmacie.**

(30) Priorité: **31.07.87 LU 86959**

(43) Date de publication de la demande:
**01.03.89 Bulletin 89/09**

(45) Mention de la délivrance du brevet:
**15.04.92 Bulletin 92/16**

(84) Etats contractants désignés:
**AT BE CH DE ES FR GB IT LI NL SE**

(56) Documents cités:
**WO-A-86/00616**

(73) Titulaire: **L'OREAL**
**14, Rue Royale**
**F-75008 Paris(FR)**

(72) Inventeur: **Maignan, Jean**
**8, Rue Halévy**
**F-93290 Tremblay les Gonesse(FR)**
Inventeur: **Restle, Serge**
**140, Rue Anatole France**
**F-93601 Aulnay sous Bois(FR)**
Inventeur: **Lang, Gérard**
**44, Avenue Lacour**
**F-95210 Saint Gratien(FR)**

(74) Mandataire: **Casalonga, Axel et al**
**BUREAU D.A. CASALONGA - JOSSE Morassistrasse 8**
**W-8000 München 5(DE)**

EP 0 304 665 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouveaux dérivés de pyrimidine oxyde-3, leur préparation et à des compositions cosmétiques ou pharmaceutiques destinées notamment à être utilisées en application topique.

On connaît déjà, dans l'état de la technique, le pipéridino-6 diamino-2,4 pyrimidine oxyde-3 ou "Minoxidil" pour ses propriétés d'agent antihypertenseur mais également pour son utilisation dans le traitement de la chute des cheveux, de la pelade, de la dermatite desquamante, de l'alopécie, etc.

La demanderesse vient de découvrir de nouveaux produits dérivés de la pyrimidine oxyde-3 qui sont des mono ou des di-urées dans les positions 2 et/ou 4 du triamino-2,4,6 pyrimidine oxyde-3.

Elle a découvert que ces produits étaient particulièrement efficaces pour la repousse des cheveux et pouvaient être utilisés notamment dans le traitement des maladies du cuir chevelu telles que la pelade, la chute des cheveux, la dermatite desquamante, l'alopécie.

L'invention a donc pour objet de nouveaux dérivés de triamino-2,4,6 pyrimidine oxyde-3.

Un autre objet de l'invention est constitué par leur procédé de préparation.

L'invention concerne également les compositions cosmétiques et/ou pharmaceutiques mettant en oeuvre ces composés.

D'autres objets de l'invention apparaîtront à la lecture de la description et des exemples qui suivent.

Les composés conformes à l'invention sont essentiellement caractérisés par le fait qu'ils répondent à la formule :

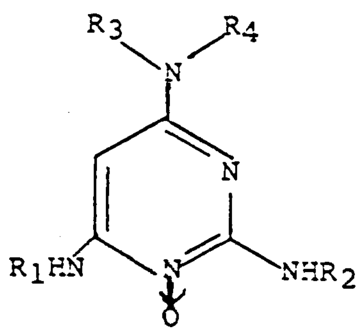

(I)

dans laquelle :

$R_3$ et $R_4$ identiques ou différents, désignent un atome d'hydrogène, un radical alkyle linéaire ou ramifié en $C_1$-$C_{18}$, alcényle ayant 2 à 18 atomes de carbone, cycloalkyle de 5 à 8 atomes de carbone, éventuellement substitués par un ou plusieurs groupements alkyle inférieur, les groupements alkyle, alcényle ou cycloalkyle pouvant eux-mêmes être substitués par un ou plusieurs groupements hydroxyle, $R_3$ et/ou $R_4$ désignent également un groupement aryle ou aralkyle répondant à la formule :

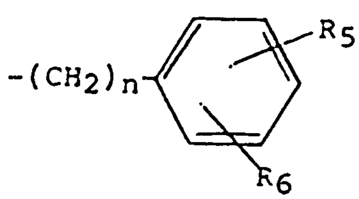

(II)

dans laquelle :

n peut prendre les valeurs de 0 à 4 et où $R_5$ et/ou $R_6$, indépendamment l'un de l'autre, désignent hydrogène, un groupement alkyle inférieur en $C_1$-$C_6$, un groupement nitro, hydroxyle, alcoxy ou un atome d'halogène, ou encore un groupement carboxylique ainsi que les formes salifiées, esters et amides de ces derniers,

$R_3$ et $R_4$ pris ensemble âvec l'atome d'azote auquel ils sort reliés, peuvent former un groupement morpholino, pipéridino, pyrrolidino, pipérazino, N-alkyl-4' pipérazino, dans lequel le groupement alkyle en position 4' contient de préférence 1 à 6 atomes de carbone dont l'un est éventuellement substitué par un groupement hydroxyle.

$R_1$ et/ou $R_2$, indépendamment l'un de l'autre, désignent un atome d'hydrogène ou un groupement carbamoyle de formule :

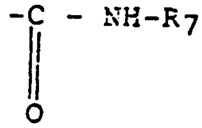

(III)

sous réserve toutefois que $R_1$ et $R_2$ ne désignent pas simultanément un atome d'hydrogène,

$R_7$ désigne, un radical alkyle, linéaire ou ramifié en $C_1$-$C_{18}$, un groupement alcényle en $C_2$-$C_{18}$, un groupement cycloalkyle en $C_5$-$C_8$,

$R_7$ peut également désigner un radical aryle ou aralkyle répondant à la formule (II) définie ci-dessus.

Les composés conformes à l'invention peuvent également exister sous la forme tautomère répondant aux formules (IA) et (IB) suivantes :

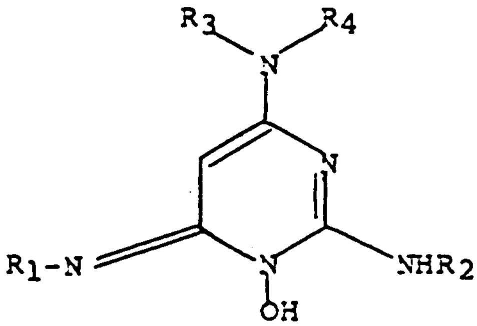

(IA)

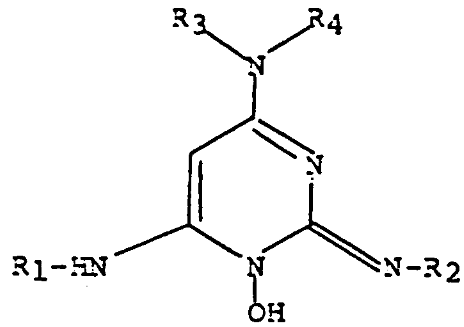

(IB)

Il est entendu que ces formes tautomères (I), (IA) et (IB) peuvent être présentes dans le mélange dans des proportions variables et l'une peut être prépondérante par rapport aux autres suivant la nature des substituants $R_3$, $R_4$, $R_1$, $R_2$ ou suivant la nature du solvant.

Conformément à l'invention, les radicaux alkyle ayant 1 à 18 atomes de carbone sont de préférence choisis parmi les radicaux méthyle, éthyle, propyle, éthyl-2 hexyle, octyle, dodécyle, hexadécyle et octadécyle.

On entend par radical alkyle inférieur un groupement ayant de 1 à 6 atomes de carbone et notamment les groupements méthyle, éthyle, isopropyle, butyle et tertiobutyle.

Les groupements alcényle préférés sont plus particulièrement les groupements allyle, buténylе, hexényle, dodécényle, hexadécényle et octadécényle.

Les radicaux aryle ou aralkyle préférés sont plus particulièrement les radicaux phényle, tolyl-4, nitro-2 phényle, nitro-4 phényle, fluoro-4 phényle, chloro-4 phényle, carboxy-2 phényle, carboxy-4 phényle, hydroxy-4 phényle, benzyle, phénéthyle.

Un atome d'halogène est de préférence un atome de chlore ou de fluor.

Les composés particulièrement préférés sont ceux dans lesquels $R_3$ et $R_4$ forment avec l'atome d'azote auquel ils sont liés un groupement pipéridino, et $R_1$ et/ou $R_2$ désignent le radical :

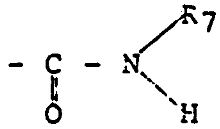

,

$R_7$ correspond à un groupe alkyle inférieur et en particulier butyle, cycloalkyle en $C_5$-$C_8$, en particulier cyclohexyl, aryle ou aralkyle, en particulier toluyle.

Les composés conformes à l'invention sont préparés, selon une première variante, suivant un procédé consistant essentiellement à traiter la chloro-6 diamino-2,4 pyrimidine de formule (IV) :

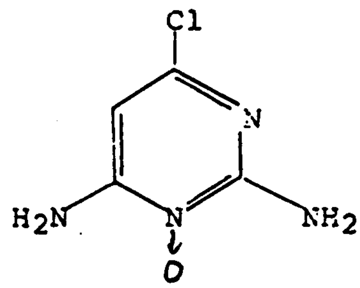

(IV)

par un isocyanate de formule :

$R_7 - N = C = O$

où $R_7$ a les significations indiquées ci-dessus, dans un solvant organique aprotique polaire, à procéder à l'oxydation des dérivés en résultant et à faire suivre d'un traitement par une amine de formule :

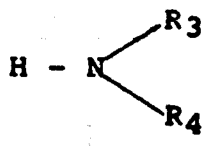

dans laquelle $R_3$ et $R_4$ ont les significations indiquées ci-dessus, pour déplacer l'atome de chlore.

Le procédé conforme à l'invention peut être illustré par les schémas réactionnels suivants :

SCHEMA A

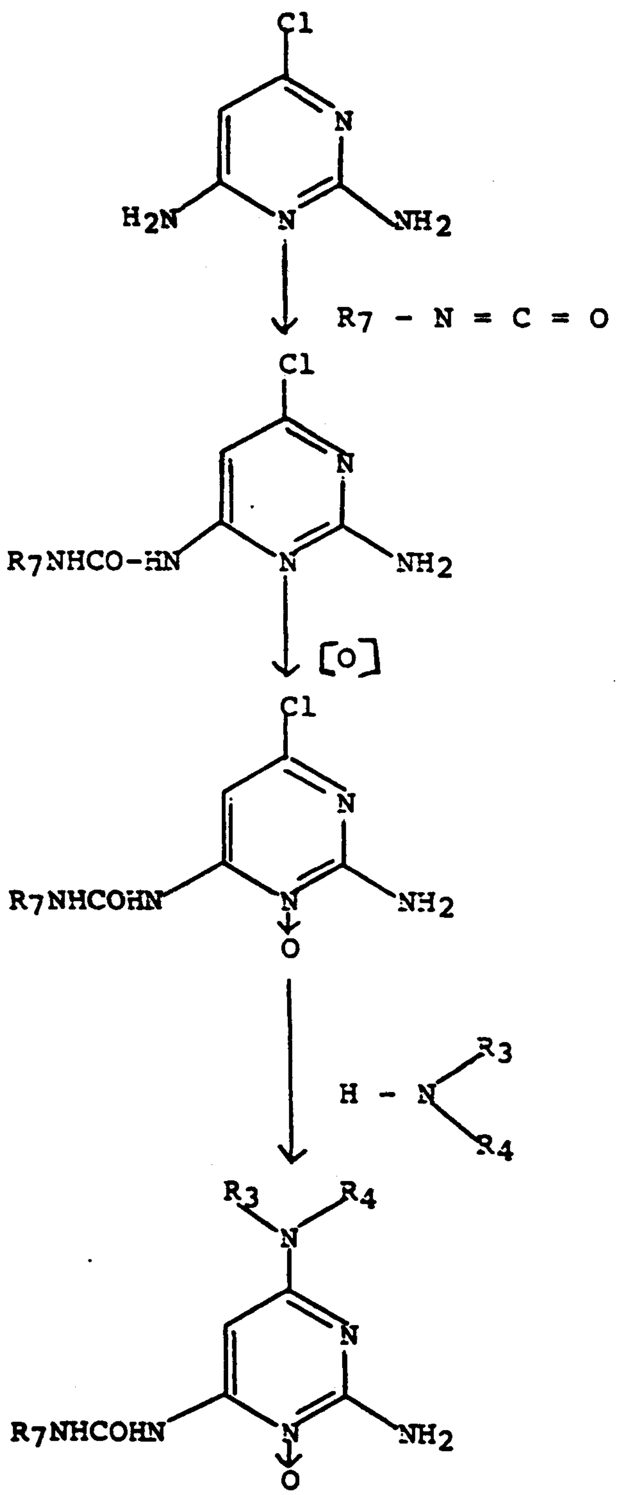


(IV)

(1)

(V)

(2)

(VII)

(3)

(VIII)

SCHEMA B

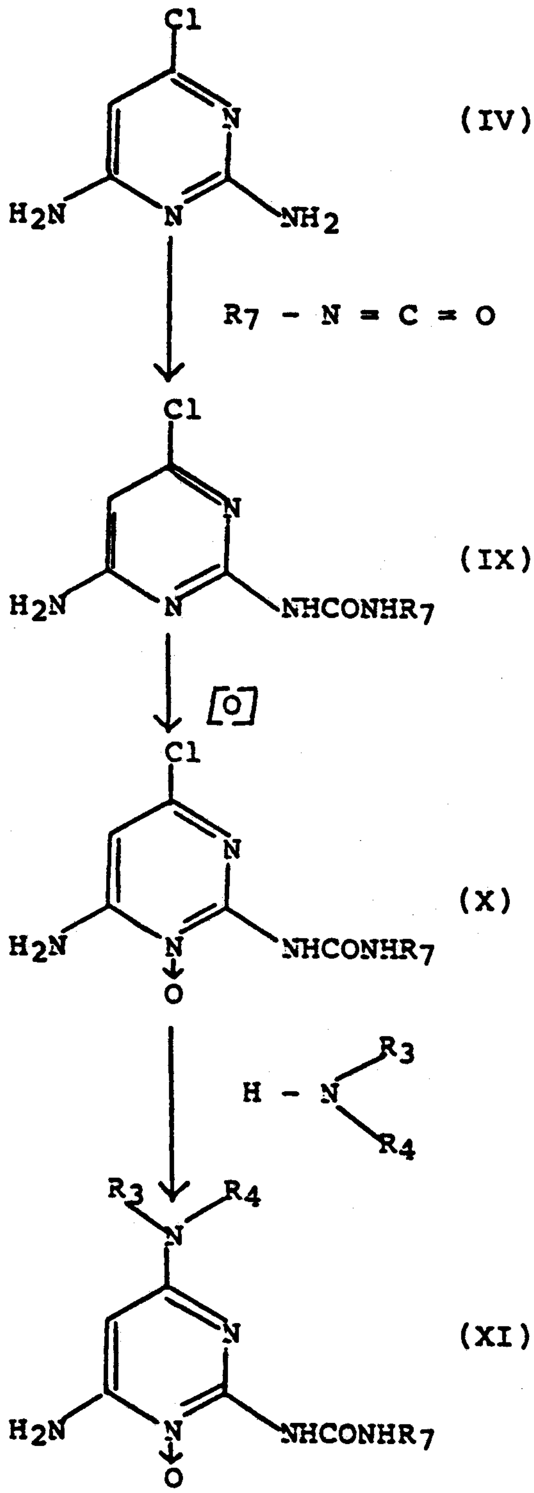


(IV)

(IX)

(X)

(XI)

SCHEMA A

L'étape 1 consiste à partir de la chloro-6 diamino-2,4 pyrimidine de formule (IV) qui est un produit commercial et à traiter ce composé par un isocyanate répondant à la formule :

$R_7 - N = C = O$

dans laquelle $R_7$ a les significations indiquées ci-dessus.

La réaction est mise en oeuvre dans un solvant organique aprotique polaire tel que par exemple le diméthylsulfoxyde (DMSO), le diméthylformamide (DMF), ou la N-méthylpyrrolidone. En utilisant un excès d'isocyanate, on obtient de façon surprenante le dérivé d'urée répondant à la formule (V) en quantité prépondérante.

En prolongeant le temps de réaction et en maintenant le produit (V) en présence d'un excès d'isocyanate, on peut synthétiser le dérivé de di-urée de formule (VI) :

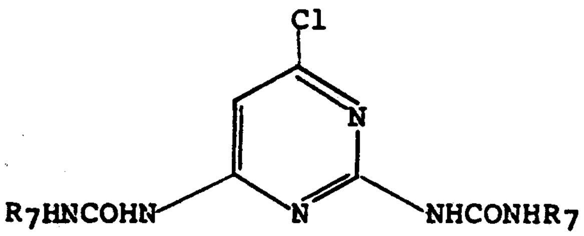

(VI)

dont les deux fonctions amino dans les positions 2 et 4 sont transformées en urée correspondante.

Dans l'étape 2, le composé de formule (V) est oxydé en un pyrimidine oxyde-3 correspondant de formule (VII), soit par action d'acide métachloroperbenzoïque ou en utilisant des peracides formés "in situ" par action de l'eau oxygénée sur un acide organique.

On obtient particulièrement de bons résultats en opérant en phase hétérogène, dans un mélange d'un solvant chloré tel que le dichlorométhane ou un éther tel que le dioxanne en présence d'environ 5 à 30% d'acide formique et de préférence 10%, contenant en particulier le composé de formule (V) auquel on ajoute un léger excès d'eau oxygénée. La réaction est conduite de préférence à une température comprise entre 0 et 70 °C. Cette réaction d'oxydation est sélective et on isole avec un très bon rendement les pyrimidines oxyde-3 de formule (VII) soit en éliminant le solvant chloré par évaporation sous vide, le mélange obtenu est versé dans l'eau et le produit précipité est isolé par filtration, soit en fonction de la nature du groupement $R_7$, directement isolé par filtration du milieu réactionnel. La pureté de ces produits est suffisante pour les traiter directement dans la troisième étape.

Dans l'étape 3, les produits obtenus dans l'étape précédente peuvent être utilisés directement et être traités par une amine de formule :

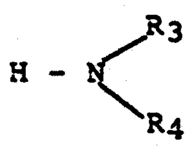

Cette réaction de déplacement du chlore des produits (VII) est réalisée suivant la basicité de l'amine :

- soit en utilisant celle-ci en excès; dans ce cas là, elle sert à la fois de solvant et de réactif;
- soit en utilisant un solvant organique et l'amine est utilisée en léger excès de 1,5 à 5 équivalents.

Cette réaction est réalisée à une température compatible avec le point d'ébullition de l'amine et est comprise entre 0 et 150 °C, mais de préférence aux environs de 70 °C. On obtient ainsi avec un bon rendement les pyrimidines oxyde de formule (VIII).

On opère de la même façon suivant les étapes 2 et 3 pour transformer les di-urées de formule (VI) en produit (XIII).

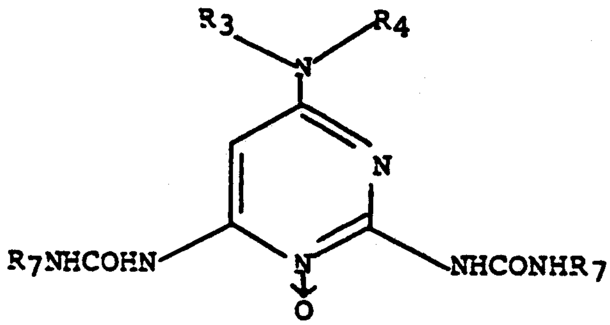

(XIII)

SCHEMA B

En utilisant un autre solvant aprotique tel que par exemple le tétrahydrofuranne (THF) au cours de la première étape de synthèse, on obtient de façon surprenante non pas l'urée de formule (V) ou alors en faible quantité, mais la chloro-6 N-carbamoylamino-2 amino-4 pyrimidine de formule (IX) suivant le schéma B.

Ce produit comme ci-dessus est alors oxydé dans les mêmes conditions opératoires en un oxyde correspondant (X) et enfin par réaction avec une amine

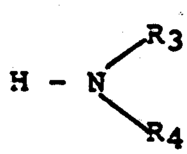

,

on obtient les produits de formule (XI)

Selon une deuxième variante, on peut obtenir les composés de formule (XI), comme illustré sur le schéma C suivant en traitant l'hydroxy-6 diamino-2,4 pyrimidine (IVb) par un isocyanate $R_7$-N=C=O dans un solvant aprotique tel que la N-méthylpyrrolidone, on obtient l'urée de formule (IXb). Cette dernière par réaction avec le chlorure de tosyle conduit au tosylate corresponsant (IXc).

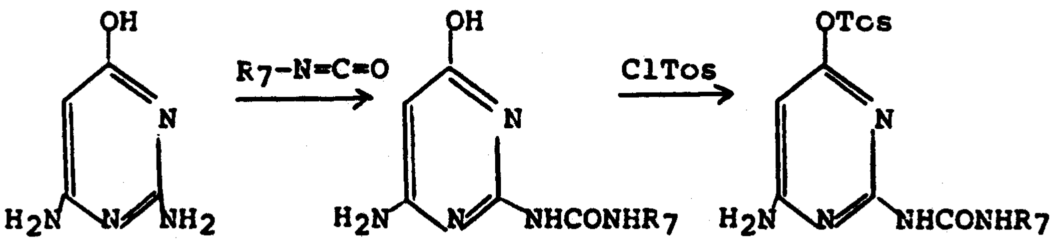


(IVb) (IXb) (IXc)

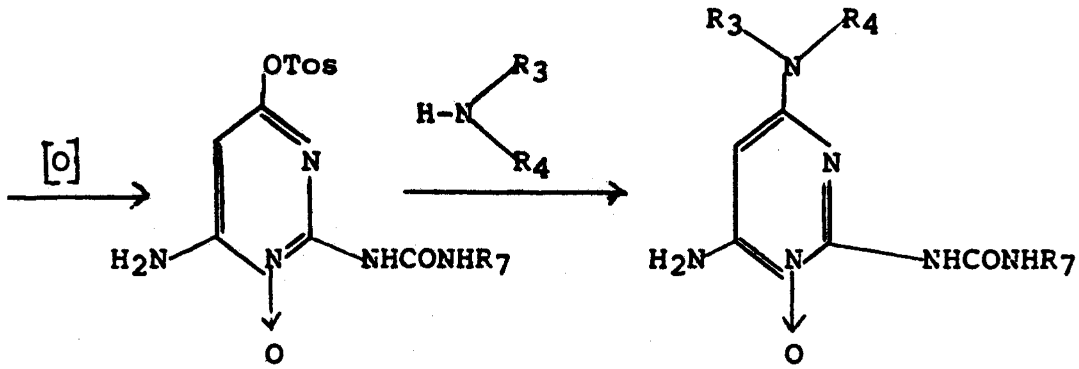


(Xc) (XI)

SCHEMA C

Le tosylate traité par un peracide organique est transformé en N-oxyde correspondant (Xc) qui par réaction avec un excès d'amine

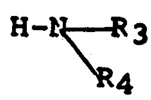

donne le composé de l'invention de formule (XI).

Selon une troisième variante, les composés conformes à l'invention peuvent également être préparés en partant des triamino-2,4,6 pyrimidine oxyde-3 en les traitant par un isocyanate, la fonction amino en position 6 étant substituée comme indiqué ci-dessus.

Les triamino-2,4,6 pyrimidine oxyde-3 sont connus en eux-mêmes et peuvent être préparés suivant le procédé décrit dans le brevet US-A-3.910.928.

L'isocyanate est mis à réagir avec le triamino-2,4,6 pyrimidine oxyde-3 selon le schéma D indiqué ci-après dans un solvant aprotique polaire tel qui le DMSO, le DMF, le tétrahydrofuranne (THF), le diméthylacétamide, etc., à une température comprise entre 20 et 100 ° C. Il est entendu que ces températures doivent être compatibles avec le point d'ébullition de l'isocyanate.

SCHEMA D

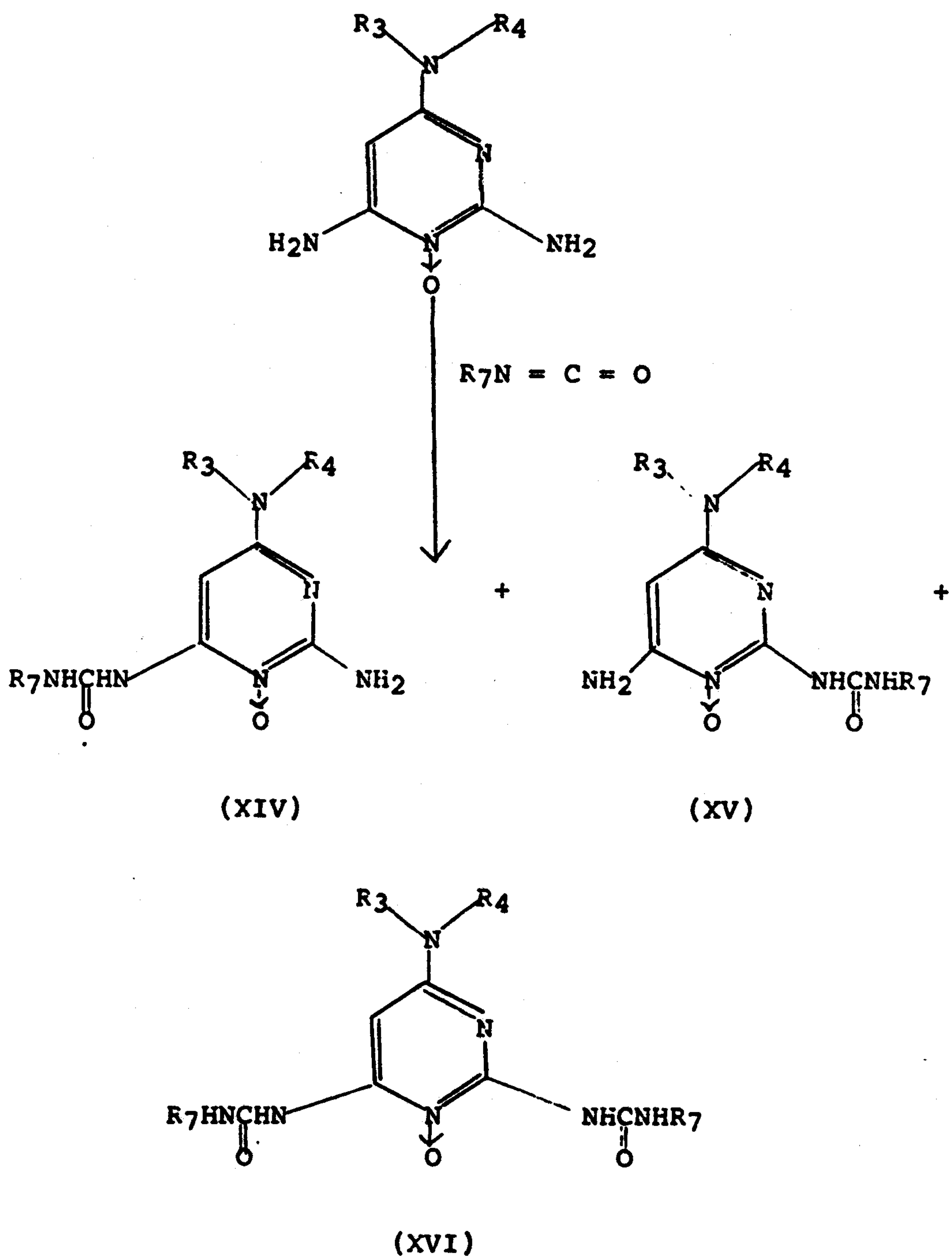


(XIV) (XV)

(XVI)

De façon générale, bien qu'en utilisant un excès d'isocyanate, la synthèse des mono-urées de formule (XIV) ou (XV) est beaucoup plus rapide que celle des di-urées de formule (XVI).

Il est donc possible d'obtenir conformément à l'invention les dérivés de monocondensation (XIV) ou (XV) avec un bon rendement. D'une façon générale, ou obtient toujours un mélange des deux produits (XIV) et (XV) qui contient, suivant les temps de réaction et la quantité d'isocyanate utilisée, une quantité plus ou moins importante de di-urée de formule (XVI).

En choisissant un solvant approprié, on obtient d'une façon surprenante, en quantité très prépondérante, l'un des produits (XIV) ou (XV). Ainsi, en opérant dans le DMSO, le produit nettement majoritaire obtenu est le composé de formule (XIV).

A partir de ces deux produits (XIV) et (XV), comme indiqué ci-dessus, on peut préparer les di-urées de formule (XVI) dont les deux fonctions urée sont identiques.

Il est possible, conformément à l'invention, de préparer également les produits de formules (XVII) ou (XVIII) dont les deux fonctions urée sont différentes :

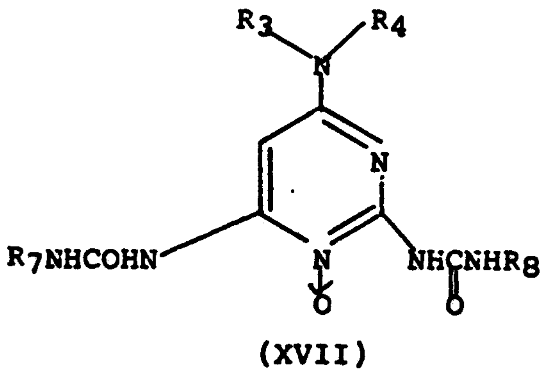

(XVII)

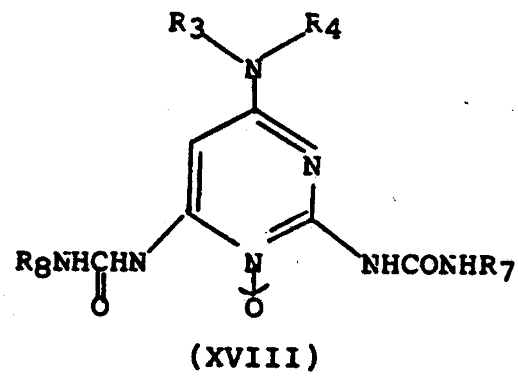

(XVIII)

en traitant respectivement les mono-urées (XIV) ou (XV) par un isocyanate de formule :

$R_8 - N = C = O$

dans lesquelles le radical $R_8$, identique ou différent de $R_7$, a les mêmes significations que le radical $R_7$.

Réciproquement, il peut être avantageux de préparer les monourées de structure (XV) à partir des diurées de formule (XVI) par un traitement de ces dernières en milieux basique.

Les composés conformes à l'invention peuvent être utilisés dans le domaine cosmétique ou pharmaceutique, notamment dans les applications topiques. La demanderesse a constaté d'une façon surprenante que les composés conformes à l'invention présentent un effet cardiovasculaire nettement inférieur à celui des composés dont la fonction amino en position 2 et/ou 4 sont libres.

Ils présentent par ailleurs une bonne stabilité.

Les compositions cosmétiques ou pharmaceutiques qui constituent un autre objet de l'invention sont utilisées notamment pour traiter le cuir chevelu et plus particulièrement la pelade, la chute des cheveux, l'alopécie, la dermatite desquamante, etc.

Ces compositions sont essentiellement caractérisées par le fait qu'elles contiennent dans un support pharmaceutiquement ou cosmétiquement approprié pour une application topique, au moins un composé répondant à la formule (I) ou aux formes tautomères de celui-ci et/ou un de ses sels, esters ou amides.

Les compositions conformes à l'invention peuvent comporter tout support approprié pour l'application topique et compatible avec la substance active, les composés pouvant se trouver dans ce support, soit à l'état dissous, soit à l'état dispersé.

Les compositions destinées à être utilisées en pharmacie se présentent sous forme d'onguents, de teintures, de crèmes, de pommades, de poudres, de timbres, de tampons imbibés, de solutions, d'émulsions, de lotions, de gels, de sprays ou de suspensions. Elles peuvent être, soit anhydres ou aqueuses selon l'indication clinique.

Les composés sont présents dans ces compositions dans des concentrations comprises entre 0,1 et 10% en poids et en particulier comprises entre 0,2 et 5% en poids.

Les compositions cosmétiques sont notamment destinées à être utilisées sous forme de lotions, de gels, de savons ou de shampooings et contiennent dans un support physiologiquement acceptable au moins un composé de formule (I) ou l'un de ses sels, esters ou amides.

La concentration des composés de formule (I) est de préférence comprise dans ces cas là entre 0,01 et 5% en poids, en particulier entre 0,05 et 3% en poids.

Les compositions conformes à l'invention peuvent contenir différents additifs habituellement utilisés en cosmétique ou en pharmacie et qui sont inertes vis-à-vis de la substance active. On peut citer à cet effet des agents hydratants comme la thiamorpholinone et ses dérivés ou l'urée; des agents anti-séborrhéiques tels que la S-carboxyméthylcystéine, la S-benzylcystéamine et leurs dérivés, la tioxolone; des agents favorisant la repousse des cheveux comme le phénytoïn (diphényl-5,5 imidazolinedione-2,4) ou encore l'iodure d'oxapropanium, l'acide rétinoïque et ses dérivés, les composés décrits dans les demandes de

brevet EP-A-0 220 118, EP-A-0 232 199, FR-A-2 600 064, FR-A-2 599 031, FR-A-2 601 002, EP-A-0 260 162 et GB-A-2 197 316, l'anthraline et ses dérivés; des agents anti-inflammatoires stéroïdiens ou non stéroïdiens; des caroténoïdes et, plus particulièrement, le $\beta$-carotène, les acides eicosatétraynoïque-5,8,11,14 et eicosa-triynoïque-5,8,11 et leurs esters et amides.

Les compositions peuvent également contenir des agents conservateurs, des agents stabilisants, des agents régulateurs de pH, des agents modificateurs de pression osmotique, des agents émulsifiants, des filtres UV-A et UV-B, des antioxydants tels que l'$\alpha$-tocophérol, le butylhydroxy-anisole ou le butylhydroxy-toluène.

L'invention a également pour objet un procédé de traitement cosmétique des cheveux consistant à appliquer sur le cuir chevelu au moins une composition telle que définie ci-dessus.

Un autre objet de l'invention est enfin constitué par l'utilisation des composés de formule (I) dans la préparation d'un médicament pour le traitement de la pelade, de la chute des cheveux, de la dermatite desquamante.

Les exemples suivants sont destinés à illustrer l'invention sans pour autant présenter un caractère limitatif.

## EXEMPLE I

### Synthèse de la N-(pipéridino-6 amino-2 oxyde-3 pyrimidinyl-4), N′-n-butyl urée

A une suspension de 10 g de pipéridino-6 diamino-2,4 pyrimidine oxyde-3 (Minoxidil) dans 75 $cm^3$ de DMSO portée à 50-60 °C, on ajoute 6,5 $cm^3$ de butyl-isocyanate. L'addition est effectuée par fractions de 1 $cm^3$ sur 3-4 heures.

Après la dernière addition, le milieu réactionnel devient homogène. On maintient le chauffage pendant environ 2 heures, puis la solution est versée sur environ 1 litre d'eau glacée additionnée d'un peu d'acide acétique. Le produit attendu cristallise. Après neutralisation de la phase aqueuse, le brut réactionnel est filtré et séché sous vide.

Le brut réactionnel est composé du produit attendu ainsi que du produit de dicondensation.

Par purification chromatographique sur gel de silice (éluant : $CH_2Cl_2$-MeOH-$NH_4OH$), on isole les deux produits.

On obtient 8,5 g de la N-(pipéridino-6 amino-2 oxyde-3 pyrimidinyl-4), N′-n-butyl urée sous forme de cristaux blancs fondant à 202-204 °C.

| Analyse élémentaire : $C_{14}H_{24}N_6O_2$ | | | | |
|---|---|---|---|---|
| | C% | H% | N% | O% |
| Calculé : | 54,50 | 7,80 | 27,27 | 10,38 |
| Trouvé : | 54,36 | 7,80 | 27,20 | 10,51 |

On obtient également 0,500 g de produit de dicondensation sous forme de cristaux blancs dont le point de fusion est de 210 °C.

| Analyse élémentaire : $C_{19}H_{33}N_7O_3$ | | | | |
|---|---|---|---|---|
| | C% | H% | N% | O% |
| Calculé : | 55,99 | 8,16 | 24,06 | 11,77 |
| Trouvé : | 56,06 | 8,19 | 24,05 | 11,90 |

## EXEMPLE II

### Synthèse de la N,N″-(pipéridino-6 oxyde-3 pyrimidinyl-2,4) bis (N′-n-butyl) urée

A une solution de 1 g de N-(pipéridino-6 amino-2 oxyde-3 pyrimidinyl-4) N′-n-butyl urée de l'exemple 1 dans 20 $cm^3$ de DMSO portée à 50-60 °C, on ajoute 1,5 $cm^3$ de n-butylisocyanate. On maintient le chauffage pendant environ 4 heures puis la solution est versée sur de l'eau glacée et filtrée. Le produit

obtenu est séché, puis recristallisé dans du méthanol. On obtient 750 mg d'un produit blanc, fondant à 208-209 ° C et qui est conforme au produit de disubstitution obtenu dans l'exemple 1.

EXEMPLE III

Synthèse de la N-(pipéridino-6 amino-2 oxyde-3 pyrimidinyl-4), N′-cyclohexyl urée

A une suspension de 10 g de pipéridino-6 diamino-2,4 pyrimidine oxyde-3 dans 75 $cm^3$ de DMSO portée à 50-60 ° C, on ajoute 9,5 $cm^3$ de cyclohexylisocyanate. L'addition est effectuée par fractions de 1 $cm^3$ sur 3-4 heures.

Après la dernière addition, le milieu réactionnel est chauffé à 60-75 ° C pendant 5-6 heures. On vérifie en chromatographie sur couche mince la disparition du produit de départ. La solution est versée sur environ 1 litre d'eau glacée additionnée d'un peu d'acide acétique. Le produit attendu cristallise. Après neutralisation de la phase aqueuse, le brut réactionnel est filtré et séché sous vide.

Le brut réactionnel est composé du produit attendu ainsi que du produit de dicondensation.

Par purification chromatographique sur gel de silice (éluant : $CH_2Cl_2$-MeOH-$NH_4OH$), on isole les deux produits.

On obtient 9,6 g de la N-(pipéridino-6 amino-2 oxyde-3 pyrimidinyl-4), N′-cyclohexyl urée sous forme de cristaux blancs fondant à 214-215 ° C après recristallisation dans le méthanol.

| Analyse élémentaire : $C_{16}H_{26}N_6O_2$ | | | | |
|---|---|---|---|---|
| | C% | H% | N% | O% |
| Calculé : | 57,46 | 7,83 | 25,13 | 9,56 |
| Trouvé : | 57,10 | 7,77 | 25,43 | 10,20 |

On obtient également 2,5 g de produit de dicondensation sous forme de cristaux blancs dont le point de fusion est de 219-220 ° C après recristallisation dans le méthanol.

| Analyse élémentaire : $C_{23}H_{37}N_7O_3$ | | | | |
|---|---|---|---|---|
| | C% | H% | N% | O% |
| Calculé : | 60,10 | 8,11 | 21,33 | 10,44 |
| Trouvé : | 60,63 | 8,35 | 20,95 | 10,40 |

EXEMPLE IV

Synthèse de la N-(pipéridino-6 amino-2 oxyde-3 pyrimidinyl-4), N′-toluyl urée

A une suspension de 1 g de pipéridino-6 diamino-2,4 pyrimidine oxyde-3 dans 50 $cm^3$ de DMSO portée à 50-60 ° C, on ajoute 1 $cm^3$ de toluylisocyanate.

Après 1 heure de chauffage à 60 ° C, le milieu réactionnel devient homogène puis un précipité blanc se forme.

Après hydrolyse, on filtre le précipité obtenu. Le brut de réaction est séché puis recristallisé dans un mélange méthyl pyrrolidone/éther diisopropylique.

On obtient 500 mg de la N-(pipéridino-6 amino-2 oxyde-3 pyrimidinyl-4), N′-toluyl urée sous forme d'une poudre blanche fondant à 227-228 ° C.

Le spectre $^1H$ RMN 80 MHz est conforme à la structure attendue et le produit est pur en HPLC.

EXEMPLE V

Synthèse de la N-(pipéridino-6 amino-2 oxyde-3 pyrimidinyl-4), N′-n-butyl urée

1ère étape

Préparation de la N-(amino-2 chloro-6 pyrimidinyl-4), N′-n-butyl urée

A une suspension de 5 g de chloro-4 diamino-2,6 pyrimidine dans 50 $cm^3$ de DMSO, on ajoute 8,6 $cm^3$ de n-butylisocyanate, puis on chauffe le milieu réactionnel à 80°C jusqu'à disparition du produit de départ en CCM.

Après hydrolyse du milieu réactionnel, le produit est extrait à l'acétate d'éthyle. La phase organique est lavée, séchée puis concentrée sous pression réduite.

On obtient 5,1 g de produit qui est purifié par recristallisation dans l'acétate d'éthyle.

La N-(amino-2 chloro-6 pyrimidinyl-4), N′-n-butyl urée cristallise sous forme de cristaux blancs fondant à 227°C.

Le spectre $^1H$ RMN 250 MHz est conforme à la structure attendue.

2ème étape

Préparation de la N-(amino-2 oxyde-3 chloro-6 pyrimidinyl-4), N′-n-butyl urée

A une suspension de 2,8 g de N-(amino-2 chloro-6 pyrimidinyl-4), N′-n-butyl urée préparée précédemment dans un mélange de 100 $cm^3$ d'alcool et 15 $cm^3$ d'eau, on ajoute 2,17 g d'acide métachloroperbenzoïque.

La température de la réaction est maintenue à 20°C et on suit son évolution en CCM.

Après consommation du produit de départ, l'alcool est évaporé sous pression réduite et le résidu est repris dans de l'eau additionnée d'hydrogénocarbonate de sodium.

Après une demi-heure d'agitation vigoureuse de la phase aqueuse, le précipité est filtré, lavé abondamment à l'eau et séché.

On obtient 2,5 g de cristaux blancs dont le point de fusion est de 195-196°C et dont le spectre $^1H$ RMN est conforme à la structure attendue.

3ème étape

Préparation de la N-(pipéridino-6 amino-2 oxyde-3 pyrimidinyl-4), N′-n-butyl urée

Une suspension de 1 g de N-(amino-2 oxyde-3 chloro-6 pyrimidinyl-4), N′-n-butyl urée, préparée précédemment dans 10 $cm^3$ de pipéridine est chauffée pendant 1 heure à 100°C.

Le milieu réactionnel est versé sur de l'eau glacée. Le précipité obtenu est filtré, séché et recristallisé dans le méthanol.

On obtient 0,950 mg de N-(pipéridino-6 amino-2 oxyde-3 pyrimidinyl-4), N′-n-butyl urée conforme au produit obtenu à l'exemple I.

EXEMPLE VI

Synthèse de la N-(pipéridino-6 amino-2 oxyde-3 pyrimidinyl-4), N′-isopropylurée.

A une suspension de 10 g de pipéridino-6 diamino-2,4 pyrimidine oxyde-3 dans 75 $cm^3$ de DMSO portée à 50-60°C, on ajoute 4,7 $cm^3$ d'isopropylisocyanate, en solution dans un peu de DMSO, au goutte à goutte. Après 4 heures de chauffage on additionne encore 2 $cm^3$ d'isopropylisocyanate et on maintient le chauffage pendant 3 heures.

Après neutralisation de l'excès d'isocyanate avec un peu d'acide acétique, le milieu réactionnel est versé sur environ 11 d'eau glacée.

Le précipité est filtré et séché sous vide. Le brut réactionnel est composé du produit attendu ainsi que du produit de dicondensation.

Par purification chromatographique sur gel de silice (éluant : $CH_2Cl_2$-MeOH-$NH_4OH$), on isole les deux produits.

On obtient 6,2 g de la N-(pipéridino-6 amino-2 oxyde-3 pyrimidinyl-4), N′-isopropyl urée sous forme de cristaux blancs fondant à 224-225°C

| Analyse élémentaire : $C_{13}H_{22}N_6O_2$ | | | | |
|---|---|---|---|---|
| | C% | H% | N% | O% |
| Calculé : | 53,04 | 7,53 | 28,55 | 10,87 |
| Trouvé : | 52,50 | 7,56 | 28,31 | 11,38 |

On obtient également 1,75 g de N,N″-(pipéridino-6 oxyde-3 pyrimidinyl-2,4)bis (N′isopropyl)urée, produit de dicondensation, sous forme de cristaux blancs, fondant à 208-209 ° C, après recristallisation dans le méthanol.

| Analyse élémentaire : $C_{17}H_{29}N_7O_3$ | | | | |
|---|---|---|---|---|
| | C% | H% | N% | O% |
| Calculé : | 53,80 | 7,70 | 25,85 | 12,65 |
| Trouvé : | 53,77 | 7,73 | 25,88 | 12,80 |

EXEMPLE VII

Synthèse de la N-(pipéridino-6 amino-4 oxyde-3 pyrimidinyl-2), N′-cyclohexylurée

1ère étape

Synthèse de la N--amino-4 hydroxy-6 pyrimidinyl-2), N′-cyclohexylurée.

A une suspension de 10 g de diamino-2,4 hydroxy-6 pyrimidine dans environ 100 $cm^3$ de N-méthyl pyrrolidone, portée à 60 ° C, on ajoute goutte à goutte une solution de 10 $cm^3$ d'isocyanate de cyclohexyle dans 50 $cm^3$ de N-méthyl pyrrolidone. A la fin de l'addition le chauffage est maintenu pendant 3 heures. Puis on rajoute 2 $cm^3$ d'isocyanate de cyclohexyle et on porte le milieu réactionnel à 90 ° C pendant 2 heures. Après vérification en chromatographie sur couche mince de la disparition totale du produit de départ, on additionne un mélange de 5cm$^3$ d'acide acétique dans 20 $cm^3$ d'eau et on abandonne le milieu réactionnel pendant une nuit à température ambiante.

La solution est versée sur 11 d'eau et on maintient l'agitation pendant environ 1 heure. Le produit attendu est filtré et après séchage on récupère 17 g d'une poudre blanche dont le point de fusion est de 236 ° C (capillaire).

| Analyse élémentaire : $C_{11}H_{17}N_5O_2$ | | | | |
|---|---|---|---|---|
| | C% | H% | N% | O% |
| Calculé : | 52,57 | 6,82 | 27,87 | 12,73 |
| Trouvé : | 51,85 | 6,75 | 27,53 | 12,55 |

2ème étape

Syntèse de la N-(paratolylsulfonyloxy-6 amino-4 pyrimidinyl-2), N′-cyclohexylurée.

A une suspension de 8 g de N-(amino-4 hydroxy-6 pyrimidinyl-2) N′-cyclohexylurée et de 12,2 g de chlorure de paratoluènesulfonyle dans un mélange de 120 $cm^3$ d'eau et 40 $cm^3$ d'acétone portée à 40 ° C, on ajoute au goutte à goutte une solution de soude 1N. On suit la réaction en contrôlant en continu le pH du milieu réactionnel pour s'assurer que la soude additionnée est rapidement consommée.

Lorsque la réaction n'évolue plus, on vérifie en chromatographie sur couche mince que tout le produit de départ a disparu et on rajoute 100 $cm^3$ de soude diluée pour éliminer l'excès de chlorure de paratoluènesulfonyle.

Le précité ainsi obtenu est filtré et lavé abondamment à l'eau (jusqu'à neutralité des eaux de rinçage.)

Après séchage on récupère 8,3 g de produit pur fondant à 203-205 ° C.

| Analyse élémentaire : $C_{18}H_{23}N_5O_4S$ | | | | | |
|---|---|---|---|---|---|
| | C% | H% | N% | O% | S% |
| Calculé : | 53,32 | 5,72 | 17,27 | 15,78 | 7,91 |
| Trouvé : | 53,11 | 5,65 | 17,31 | 15,63 | 7,88 |

3ème étape

Synthèse de la N-(paratolylsulfonyloxy-6 amino-4 oxyde-3 pyrimidinyl-2),N'-cyclohexylurée.

A une suspension de 3 g de N-(paratolylsulfonyloxy-6 amino-4 pyrimidinyl-2), N'-cyclohexylurée dans 100 $cm^3$ de dioxanne on ajoute 20 $cm^3$ d'acide formique et 6 $cm^3$ d'eau oxygénée à 110 volumes et on porte le milieu réactionnel à 55°C pendant environ 1 heure (le produit de départ passe en solution). On rajoute 3 $cm^3$ d'eau oxygénée à 110 volumes et on maintient le chauffage pendant 1 heure.

Le mélange réactionnel est versé sur 300 $cm^3$ d'eau glacée et le précipité qui se forme est filtré et lavé abondamment à l'eau.

Après séchage on récupère une poudre blanche dont le spectre $^1H$ RMN 80MHz est conforme à la structure attendue et dont le point de fusion est de 215°C. (le produit commence à brunir à partir de 130°C).

4ème étape

Synthèse de la N-(pipéridino-6 amino-4 oxyde -3 pyrimidinyl-2), N'-cyclohexylurée.

A une solution de 1,5g de N-(paratolylsulfonyloxy-6 amino-4 oxyde-3 pyrimidinyl-2), N'-cyclohexylurée dans 50 $cm^3$ de THF on ajoute 0,5 $cm^3$ de pipéridine. On agite le milieu réactionnel pendant une 1/2 heure à température ambiante, puis on chauffe à 60°C pendant 1 heure.

A la fin de la réaction, le milieu est versé dans l'eau et le produit est extrait à l'acétate d'éthyle.

Le brut de réaction est purifié par chromatographie sur gel de silice (éluant : $CH_2Cl_2$-MeOH) et on récupère 750 mg d'une poudre blanche légèrement rosée qui fond à 183-185°C, dont le spectre $^1H$ RMN 80 MHz correspond à la structure attendue.

EXEMPLE VIII

Autre synthèse de la N-(pipéridino-6 amino-4 oxyde-3 pyrimidinyl-2), N'-cyclohexylurée.

A une solution de 1 g de N,N''-(pipéridino-6 oxyde-3 pyrimidinyl-2,4) bis (N'-cyclohexyl)urée dans 50 $cm^3$ d'isopropanol on ajoute 30 $cm^3$ d'une solution aqueuse de potasse 10N et on porte le mélange réactionnel à 80°C pendant 4 heures.

Après vérification de la disparition totale du produit de départ on évapore l'isopropanol et le résidu est repris avec 100 $cm^3$ d'eau.

Le produit attendu est extrait à l'acétate d'éthyle, la phase organique est abondamment lavée, puis séchée sur sulfate de magnésium.

Après évaporation du solvant ou récupère 500 mg d'un produit blanc qui recristallise dans un mélange méthanol-eau et dont le point de fusion est de 180-182°C.

EXEMPLE IX

Synthèse de la N-(pipéridino-6 amino-4 oxyde -3 pyrimidinyl-2), N'-n-butylurée.

1ère étape

Synthèse de la N-(amino-4 hydroxy-6 pyrimidinyl-2), N'-n-butylurée.

A une suspension de 25 g de diamino-2,4 hydroxy-6 pyrimidine dans environ 200 $cm^3$ de N-méthyl pyrrolidone portée à 60°C on ajoute en une fois 24,6 $cm^3$ de n-butylisocyanate. La réaction est

exothermique, la température monte à 100 ° C. On maintient la température à 80 ° C pendant 1 heure. La solution est jaune limpide et en chromatographie sur couche mince le produit de départ a entièrement disparu.

On laisse revenir le milieu réactionnel à température ambiante et on ajoute 20 $cm^3$ d'acide acétique dans 50 $cm^3$ d'éthanol pour détruire l'excès de n-butylisocyanate.

Puis la solution est versée sur 1,51 d'eau glacée, l'agitation est maintenue pendant 30 minutes et le précipité est filtrée.

Après séchage on récupère 40,4 g de cristaux blancs dont le point de fusion est de 226 ° C et dont le spectre $^1H$ RMN 80 MHz est conforme à la structure attendue.

| Analyse élémentaire : $C_9H_{15}N_5O_2$ | | | | |
|---|---|---|---|---|
| | C% | H% | N% | O% |
| Calculé : | 47,99 | 6,71 | 31,09 | 14,21 |
| Trouvé : | 47,54 | 6,65 | 29,75 | 14,04 |

2ème étape

Synthèse de la N-(paratolylsulfonyloxy-6 amino-4 pyrimidinyl-2), N′-n-butylurée.

A une suspension de 30 g de N-(amino-4 hydroxy-6 pyrimidinyl-2), N′-n-butylurée et de 50,7 g de chlorure de paratoluène sulfonyle dans un mélange de 600 $cm^3$ d'eau et de 180 $cm^3$ d'acétone portée à 40 ° C, on ajoute au goutte à goutte une solution de soude 1N.

On suit la réaction en contrôlant en continu le pH du milieu réactionnel pour s'assurer que la soude additionnée est rapidement consommée.

Lorsque la réaction n'évolue plus, on vérifie en chromatographie sur couche mince que tout le produit de départ a disparu et on rajoute 200 $cm^3$ de soude diluée pour éliminer l'excès de chlorure de paratoluènesulfonyle.

Le précipité ainsi obtenu est filtré et lavé abondamment à l'eau (jusqu'à neutralité des eaux de rinçage).

Après séchage on récupère 40,5 g d'un produit légèrement jaunâtre dont le point de fusion est de 195-197 ° C et dont le spectre $^1H$ RMN 80 MHz est conforme à la structure attendue.

EXEMPLE X

Synthèse de la N-(pipéridino-6 amino-4 oxyde-3 pyrimidinyl-2) N′-n-butylurée.

A une solution de 50 g de N,N″- (pipéridino-6 oxyde-3 pyrimidinyl-2,4) bis (N′-n-butyl) urée dans 1,2 l d'éthanol porté à ébullition sous agitation on ajoute par petites quantités 12 g de potasse. Lorsque toute la potasse introduite est solubilisée on maintient ce mélange réactionnel à une température comprise entre 70 ° et 80 ° C pendant cinq heures. La transformation de cette di-urée en monourée est suivie en chromatographie couche mince, lorsque celle-ci est complète, l'éthanol est éliminé par évaporation sous vide. Le produit est alors agité dans 250 $cm^3$ d'eau, on obtient après filtration et séchage 36 g d'un solide beige.

Après deux recristallisations dans l'éthanol on isole 25 g de N- (pipéridino-6 amino-4 oxyde-3 pyrimidinyl-2) N′-n- butylurée sous forme de cristaux blancs dont le point de fusion est de 208 ° C.

Ce produit est analysé sous forme hydratée.

| Analyse élémentaire : $C_{14}H_{24}N_6O_2,H_2O$ | | | | |
|---|---|---|---|---|
| | C% | H% | N% | O% |
| Calculé : | 53,74 | 7,89 | 26,86 | 11,51 |
| Trouvé : | 53,74 | 7,95 | 26,83 | 11,48 |

EXEMPLE XI

Synthèse de la N-(morpholino-6 amino-2 oxyde-3 pyrimidinyl-4), N′-n-butylurée.

A une suspension de 10 g de N-(amino-2 oxyde-3 chloro-6 pyrimidinyl-4), N′-n-butylurée, préparée suivant l'exemple V, dans 100 $cm^3$ d'éthanol, on ajoute 20 $cm^3$ de morpholine. On chauffe le milieu réactionnel à 50 °C pendant 4 heures, puis on évapore l'alcool. Le résidu est recristallisé deux fois dans l'acétonitrile. On obtient 6,5 g d'un produit blanc qui fond à 185-186 °C. Par traitement de ce produit sous forme de chlorhydrate avec une solution aqueuse d'hydrogénocarbonate de sodium en phase hétérogène, on obtient 4 g d'une poudre blanche fondant à 168-170 °C et dont le spectre $^1H$ RMN 80 MHz est conforme à la structure attendue.

EXEMPLE XII

Préparation du N-(pyrrolidino-6 amino-2 oxyde-3 pyrimidinyl-4) N′-n- butylurée et du :

N,N″-(pyrrolidino-6 oxyde-3 pyrimidinyl-2,4), bis N′-n butylurée.

A une suspension de 4 g de pyrrolidino-6 diamino-2,4 pyrimidine oxyde-3 dans 40 $cm^3$ de diméthylsulfoxyde anhydre, agitée à 60 °C sous atmosphère inerte, ou ajoute 3,3 $cm^3$ de butylisocyanate au goutte à goutte. Trois heures plus tard, le produit de départ n'étant pas entièrement transformé on ajoute 0,5 $cm^3$ de butylisocyanate supplémentaire et on porte la température du mélange pendant trois heures supplémentaires à 90 °C. Ensuite, on amène la température du mélange à 40 °C et on le verse dans 300 $cm^3$ d'eau glacée. Le précipité formé est extrait deux fois à l'aide de 100 $cm^3$ d'acétate d'éthyle, la phase organique est lavée à l'eau, séchée sur sulfate de sodium et le solvant est éliminé par évaporation sous vide. Le mélange de monourée et de diurée est solubilisé dans le minimum de méthanol puis déposé sur une colonne de gel de silice. En éluant cette colonne au chlorure de méthylène puis au mélange chlorure de méthylène-méthanol on obtient des fractions contenant l'un des produits purs. Après évaporation de ces fractions on isole 2 g de N- (pyrrolidino-6 amino-2 oxyde-3 pyrimidinyl-4) N′-n-butylurée qui sont recristallisés dans l'acétate d'éthyle. Ce sont des cristaux blancs de point de fusion 191 °C.

| Analyse élémentaire : $C_{13}H_{22}N_6O_2$ | | | | |
|---|---|---|---|---|
| | C% | H% | N% | O% |
| Calculé : | 53,04 | 7,53 | 28,55 | 10,87 |
| Trouvé : | 52,66 | 7,52 | 28,29 | 10,57 |

et 0,75g de N,N"- (pyrrolidino-6 oxyde-3 pyrimidinyl-2,4), bis N'-n butylurée recristallisé dans le méthanol.

| Analyse élémentaire : $C_{18}H_{31}N_7O_3$ | | | | |
|---|---|---|---|---|
| | C% | H% | N% | O% |
| Calculé : | 54,94 | 7,94 | 24,92 | 12,20 |
| Trouvé : | 54,84 | 7,88 | 24,91 | 12,38 |

EXEMPLES DE COMPOSITIONS

1) On prépare une lotion de composition suivante :
N-(pipéridino-6 amino-2 oxyde-3 pyrimidinyl-4)
N′-n-butylurée 5 g
Propylène glycol 20 g
Alcool éthylique 50 g
Eau q.s.p. 100 g
2) On prépare la lotion destinée à stimuler la repousse des cheveux de la composition suivante :
N-(pipéridino-6 amino-2 oxyde-3 pyrimidinyl-4)

N'-cyclohexylurée 3 g
Propylène glycol 20 g
Ethanol 50 g
Eau q.s.p. 100 g

1 à 2 ml de ces lotions sont appliqués sur les zones alopéciques du cuir chevelu; ces applications, éventuellement accompagnées par un massage pour favoriser la pénétration, étant effectuées une ou deux fois par jour.

3) On prépare la concentration suivante sous forme de gel :
N-(pipéridino-6 amino-2 oxyde-3 pyrimidinyl-4)
N'-isopropylurée 2 g
Hydroxypropylcellulose vendue par la société HERCULES sous la dénomination commerciale "KLUCEL G" 3 g
Propylène glycol 20 g
Ethanol 50 g
Conservateur q.s.
Eau q.s.p. 100 g

4) On prépare la composition suivante sous forme de gel :
N'(pipéridino-6 amino-4 oxyde-3 pyrimidinyl-2)
N'-n-butylurée 3 g
Méthylhydroxypropylcellulose vendue par la société DOW CHEMICAL sous la dénomination commerciale "METHOCEL F" 1 g
Ethanol 40 g
Eau q.s.p. 100 g

5) On prépare la composition suivante sous forme de lotion :
N-(morpholino-6 amino-2 oxyde-3 pyrimidinyl-4)
N'-n-butylurée 5 g
Propylène glycol monométhyléther 30 g
Ethanol 40 g
Eau q.s.p. 100 g

## Revendications

**Revendications pour les Etats contractants suivants : AT, BE, CH, DE, FR, GB, IT, LI, NL, SE**

**1.** Composé caractérisé par le fait qu'il répond à la formule :

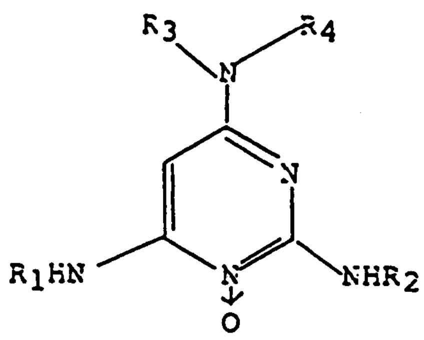

(I)

dans laquelle $R_3$ et $R_4$ identiques ou différents, désignent un atome d'hydrogène, un radical alkyle linéaire ou ramifié en $C_1$-$C_{18}$, alcényle ayant 2 à 18 atomes de carbone, cycloalkyle de 5 à 8 atomes de carbone, éventuellement substitués par un ou plusieurs groupements alkyle en $C_1$-$C_6$, les groupements alkyle, alcényle ou cycloalkyle pouvant eux-mêmes être substitués par un ou plusieurs groupements hydroxyle, $R_3$ et/ou $R_4$ désignent également un groupement aryle ou aralkyle répondant à la formule :

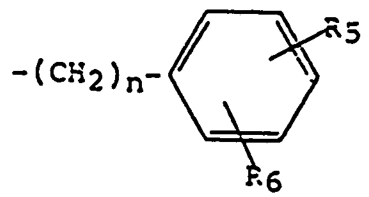

(II)

dans laquelle n peut pendre les valeurs de 0 à 4 et où $R_5$ et/ou $R_6$, indépendamment l'un de l'autre, désignent hydrogène, un groupement alkyle en $C_1$-$C_6$, un groupement nitro, hydroxyle, alcoxy ou un atome d'halogène ou encore un groupement carboxylique ainsi que les formes salifiées, esters et amides de ces derniers; $R_3$ et $R_4$ pris ensemble avec l'atome d'azote auquel ils sont reliés, peuvent former un groupement morpholino, pipéridino, pyrrolidino, pipérazino, N-alkyl-4' pipérazino;
$R_1$ et/ou $R_2$, indépendamment l'un de l'autre, désignent un hydrogène ou un groupement carbamoyle de formule :

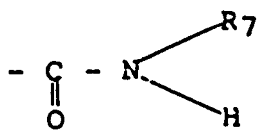

(III)

sous réserve toutefois que $R_1$ et $R_2$ ne désignent pas simultanément un atome d'hydrogène, et $R_7$ désigne un radical alkyle, linéaire ou ramifié en $C_1$-$C_{18}$, un groupement alcényle en $C_2$-$C_{18}$, un groupement cycloalkyle en $C_5$-$C_8$, $R_7$ peut également désigner un radical aryle ou aralkyle répondant à la formule (II) définie ci-dessus ainsi que leur forme tautomère répondant aux formules :

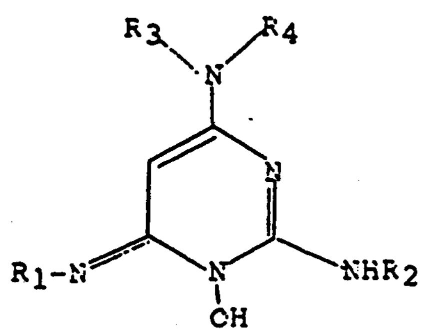

(IA)

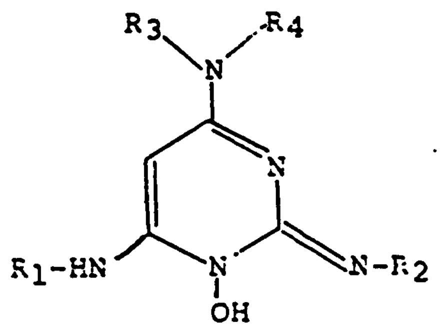

(IB)

2. Composé selon la revendication 1, caractérisé par le fait que dans la formule (I), le radical alkyle en $C_1$-$C_{18}$ est choisi parmi les radicaux méthyle, éthyle, propyle, éthyl-2 hexyle, octyle, dodécyle, hexadécyle et octadécyle, que le radical alkyle en $C_1$-$C_6$ est choisi parmi les groupements méthyle, éthyle, isopropyle, butyle et tertiobutyle, que les groupements alcényle en $C_2$-$C_{18}$ sont choisis parmi les groupements allyle, butényle, hexényle, dodécényle, hexadécényle et octadécényle, que les groupements aryle ou aralkyle sont choisis parmi les radicaux phényle, tolyl-4, nitro-2 phényle, nitro-4 phényle, fluoro-4 phényle, chloro-4 phényle, carboxy-2 phényle, carboxy-4 phényle, hydroxy-4 phényle, benzyle, phénéthyle; que l'atome d'halogène est un atome de chlore.

3. Composé selon la revendication 1 ou 2, caractérisé par le fait qu'il répond à la formule :

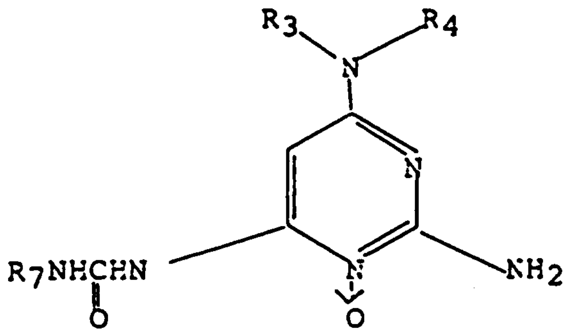

(XIV)

dans laquelle $R_3$, $R_4$ et $R_7$ ont la signification indiquée dans la revendication 1.

**4.** Composé selon la revendication 1 ou 2, caractérisé par le fait qu'il répond à la formule :

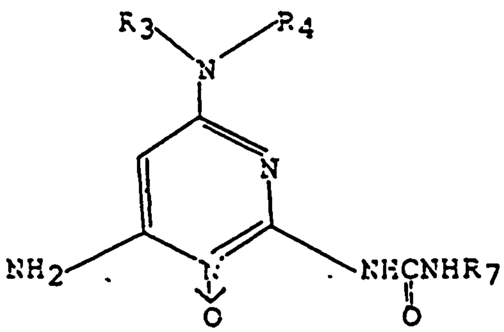

(XV)

dans laquelle $R_3$, $R_4$ et $R_7$ ont la signification indiquée dans la revendication 1.

**5.** Composé selon la revendication 1 ou 2, caractérisé par le fait qu'il répond à la formule :

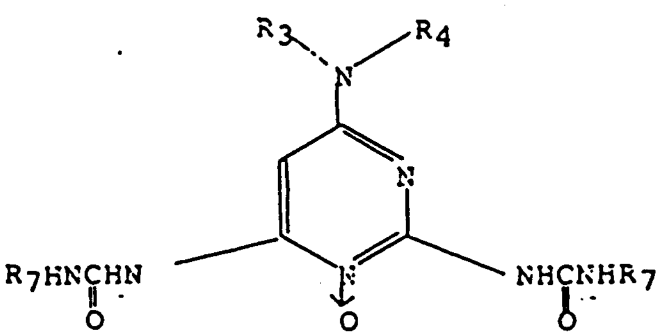

(XVI)

dans laquelle $R_3$, $R_4$, $R_7$ ont la signification indiquée dans la revendication 1.

**6.** Composé selon la revendication 1 ou 2, caractérisé par le fait que le composé répond aux formules :

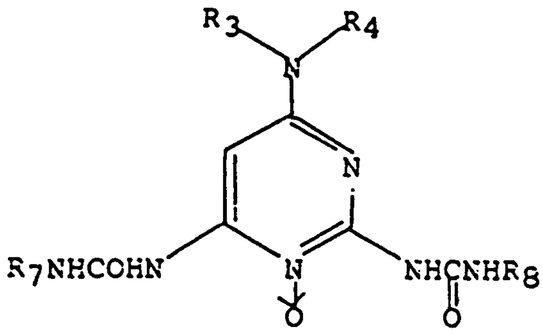

(XVII)

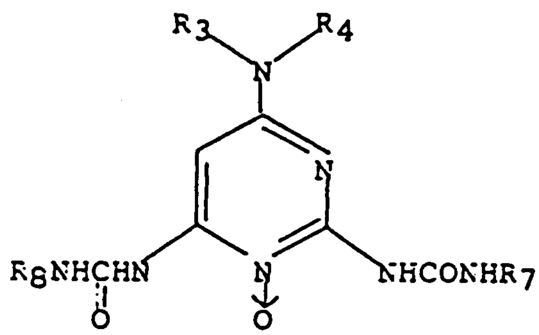

(XVIII)

dans lesquelles $R_3$, $R_4$, $R_7$ ont les significations indiquées dans la revendication 1 et $R_8$, identique ou différent de $R_7$, a les mêmes significations que $R_7$.

**7.** Composé selon l'une quelconque des revendications 1 à 6, caractérisé par le fait que les groupements $R_3$ et $R_4$ forment avec l'atome d'azote auquel ils sort liés un groupement pipéridino et que $R_1$ et/ou $R_2$ désignent hydrogène, et $R_7$ et $R_8$ désignent un groupement alkyle inférieur, cycloalkyle en $C_5$-$C_8$, aryle ou aralkyle.

**8.** Procédé de préparation du composé selon la revendication 1 ou 2, on particulier des composés répondant à la formule (VIII) :

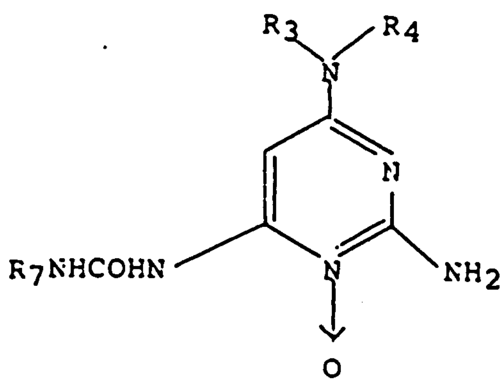

(VIII)

ou (XIII) :

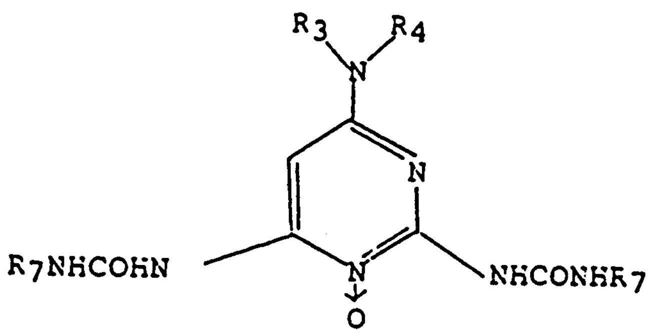

(XIII)

dans laquelle $R_3$, $R_4$ et $R_7$ ont les mêmes significations que celles indiquées dans la revendication 1, caractérisé par le fait que l'on traite la chloro-6 diamino-2,4 pyrimidine par un isocyanate de formule $R_7$-N = C = O, dans laquelle $R_7$ a les significations indiquées dans la revendication 1, dans un solvant organique aprotique polaire, que l'on procède ensuite à l'oxydation des dérivés en résultant et que l'on fait suivre par un traitement par une amine répondant à la formule

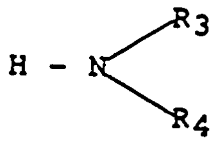

dans laquelle $R_3$ et $R_4$ ont les significations indiquées dans la revendication 1.

**9.** Procédé de préparation des composés selon la revendication 1 ou 2, en particulier des composés répondant à la formule :

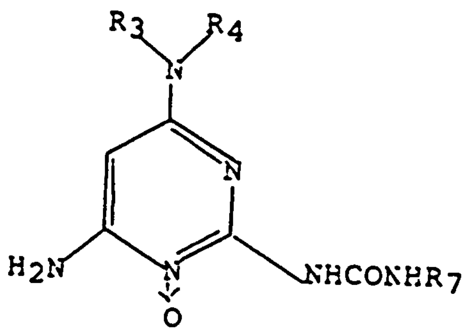

(XI)

dans laquelle $R_3$, $R_4$, $R_7$ ont les significations indiquées dans la revendication 1, en traitant la chloro-6 diamino-2,4 pyrimidine avec un isocyanate de formule $R_7$-N = C = O, dans le tétrahydrofuranne, où $R_7$ a les significations indiquées dans la revendication 1. Le produit de condensation de l'isocyanate sur l'hydroxy-6 diamino-2,4 pyrimidine obtenu dans la N-méthyl pyrrolidone est alors transformé en tosylate correspondant, ce dernier ou le dérivé de condensation de l'isocyanate sur la chloro-6 diamino-2,4 pyrimidine étant oxydé en N-oxydes correspondants que l'on fait réagir sur une amine de formule :

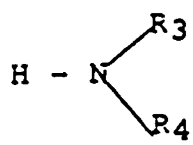

dans laquelle $R_3$ et $R_4$ ont les significations indiquées ci-dessus.

**10.** Procédé de préparation des composés selon la revendication 1 ou 2, en particulier des composés répondant aux formules (XIV), (XV) et (XVI) :

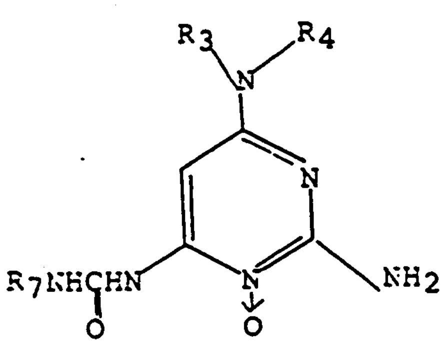

(XIV)

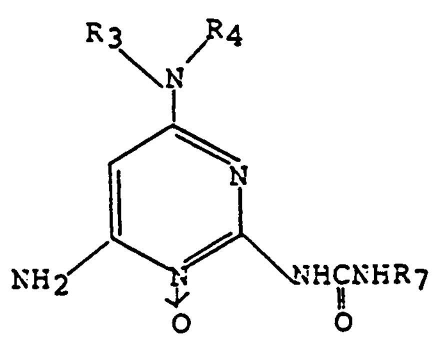

(XV)

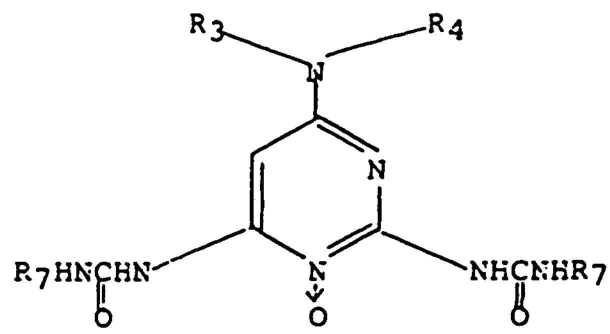

(XVI)

caractérisé par le fait que l'on traite un triamino-2,4,6 pyrimidine oxyde-3 par un isocyanate répondant à la formule $R_7$-N = C = O, et dans laquelle le groupement amino en position 6 désigne le groupement

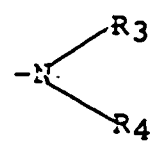

où $R_3$ et $R_4$ ont les significations indiquées ci-dessus, dans un solvant aprotique polaire à une température comprise entre 20 et 100 ° C.

**11.** Procédé selon la revendication 10, caractérisé par le fait que l'on fait réagir les mono-urées de formule (XIV) ou (XV) avec un isocyanate de formule $R_8$ - N = C = O :

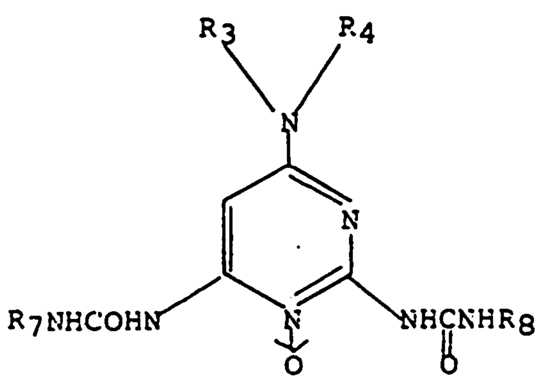

(XVII)

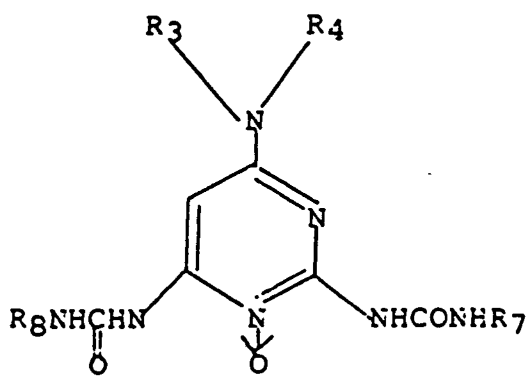

(XVIII)

dans lesquelles $R_3$, $R_4$, et $R_7$, ont les significations indiquées dans la revendication 1 et $R_8$, identique ou différent de $R_7$, a les mêmes significations que $R_7$.

**12.** Composition cosmétique ou pharmaceutique, caractérisée par le fait qu'elle contient dans un support approprié pour une application topique au moins un composé répondant à l'une quelconque des revendications 1 à 7.

**13.** Composition pharmaceutique selon la revendication 12, caractérisée par le fait qu'elle se présente sous forme d'onguents, de teintures, de crèmes, de pommades, de poudres, de timbres, de tampons imbibés, de solutions, d'émulsions, de lotions, de gels, de sprays ou de suspensions anhydres ou aqueuses, contenant au moins un composé tel que défini dans l'une quelconque des revendications 1 à 7.

**14.** Composition selon la revendication 13, caractérisée par le fait que les composés de formule (I) sont

présents dans des concentrations comprises entre 0,1 et 10% en poids par rapport au poids total de la composition, en particulier entre 0,2 et 5% en poids.

**15.** Composition cosmétique selon la revendication 12 sous forme de lotions, de gels, de savons, de shampooings, caractérisée par le fait qu'elle contient dans un support physiologiquement acceptable au moins l'un des composés tels que définis dans l'une quelconque des revendications 1 à 7 à une concentration comprise entre 0,01 et 5% en poids.

**16.** Composition selon la revendication 15, caractérisée par le fait qu'elle contient en plus des agents hydratants, des agents antiséborrhéiques, des agents favorisant la repousse des cheveux, des agents anti-inflammatoires, stéroïdiens ou non stéroïdiens, des caroténoïdes, les acides eicosatétraynoïque-5,8,11,14 et eicosatriynoïque-5,8,11 et leurs esters et amides.

**17.** Composition selon l'une quelconque des revendications 15 à 16, caractérisée par le fait qu'elle contient également des agents conservateurs, des agents stabilisants, des agents régulateurs de pH, des agents modificateurs de pression osmotique, des agents émulsifiants, des filtres UV-A et UV-B, des agents antioxydants.

**18.** Procédé de traitement cosmétique des cheveux, caractérisé par le fait que l'on applique sur le cuir chevelu au moins une composition telle que définie dans l'une quelconque des revendications 14 à 17.

**19.** Utilisation des composés selon l'une quelconque des revendications 1 à 7 pour la préparation d'un médicament pour le traitement de la pelade, de la chute des cheveux, de la dermatite desquamante.

**Revendications pour l'Etat contractant suivant : ES**

**1.** Procédé de préparation du composé qui répond à la formule :

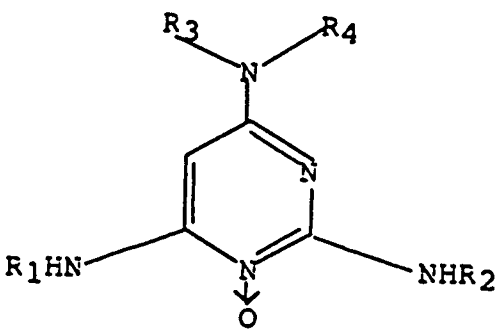

(I)

dans laquelle $R_3$ et $R_4$, identiques ou différents, désignent un atome d'hydrogène, un radical alkyle linéaire ou ramifié en $C_1$-$C_{18}$, alcényle ayant 2 à 18 atomes de carbone, cycloalkyle de 5 à 8 atomes de carbone, éventuellement substitués par un ou plusieurs groupements alkyle en $C_1$-$C_6$ , les groupements alkyle, alcényle ou cycloalkyle pouvant eux-mêmes être substitués par un ou plusieurs groupements hydroxyle, $R_3$ et/ou $R_4$ désignent également un groupement aryle ou aralkyle répondant à la formule :

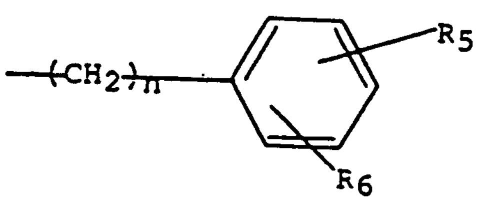

(II)

dans laquelle n peut prendre les valeurs de 0 à 4 et où $R_5$ et/ou $R_6$, indépendamment l'un de l'autre, désignent hydrogène, un groupement alkyle en $C_1$-$C_6$, un groupement nitro, hydroxyle, alcoxy ou un atome d'halogène ou encore un groupement carboxylique ainsi que les formes salifiées, esters et

amides de ces derniers; $R_3$ et $R_4$ pris ensemble avec l'atome d'azote auquel ils sont reliés, peuvent former un groupement morpholino, pipéridino, pyrrolidino, pipérazino, N-alkyl-4' pipérazino;
$R_1$ et/ou $R_2$, indépendamment l'un de l'autre, désignent un hydrogène ou un groupement carbamoyle de formule :

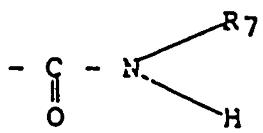

(III)

sous réserve toutefois que $R_1$ et $R_2$ ne désignent pas simultanément un atome d'hydrogène, et $R_7$ désigne un radical alkyle, linéaire ou ramifié en $C_1$-$C_{18}$, un groupement alcényle en $C_2$-$C_{18}$, un groupement cycloalkyle en $C_5$-$C_8$, $R_7$ peut également désigner un radical aryle ou aralkyle répondant à la formule (II) définie ci-dessus ainsi que leur forme tautomère répondant aux formules :

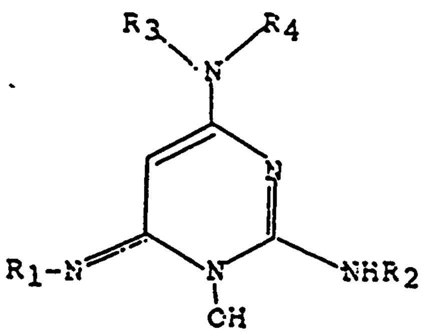

(IA)

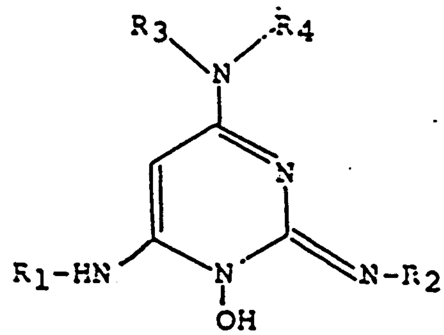

(IB)

caractérisé par le fait que :

(A) pour préparer des composés répondant à la formule (VIII) :

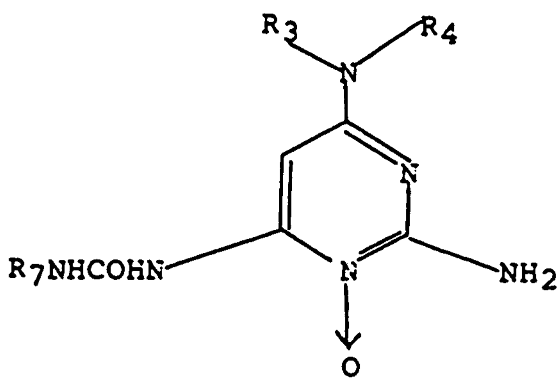

(VIII)

ou (XIII) :

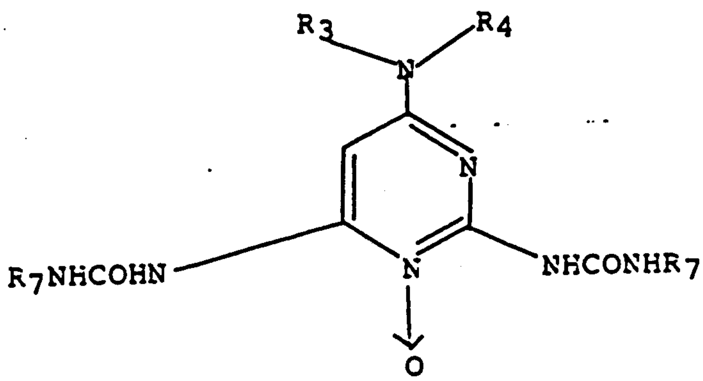

(XIII)

dans laquelle $R_3$, $R_4$ et $R_7$ ont les mêmes significations que celles indiquées ci-dessus, l'on traite la chloro-6 diamino-2,4 pyrimidine par un isocyanate de formule $R_7$-N = C = O, dans laquelle $R_7$ a les significations indiquées ci-dessus, dans un solvant organique aprotique polaire, que l'on procède ensuite à l'oxydation des dérivés en résultant et que l'on fait suivre par un, traitement par une amine répondant à la formule :

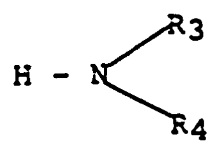

dans laquelle $R_3$ et $R_4$ ont les significations indiquées ci-dessus;

(B) pour préparer des composés répondant à la formule :

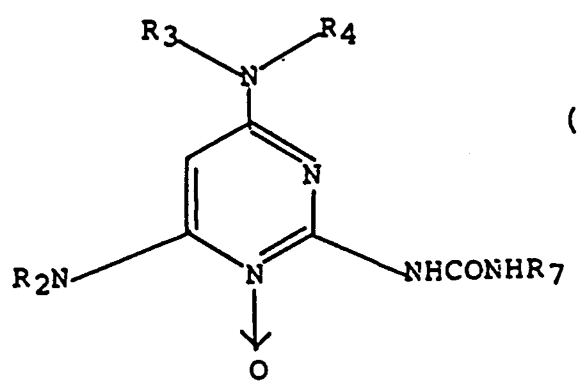

(XI)

dans laquelle $R_3$, $R_4$, $R_7$ ont les significations indiquées ci-dessus, l'on traite la chloro-6 diamino-2,4 pyrimidine avec un isocyanate de formule $R_7$-N = C = O, dans le tétrahydrofuranne, où $R_7$ a les significations indiquées ci-dessus. Le produit de condensation de l'isocyanate sur l'hydroxy-6 diamino-2,4 pyrimidine obtenu dans la N-méthylpyrrolidone est alors transformé en tosylate correspondant, ce dernier ou le dérivé de condensation de l'isocyanate sur la chloro-6 diamino-2,4 pyrimidine étant oxydé en N-oxydes correspondants que l'on fait réagir sur une amine de formule :

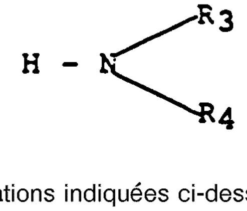

dans laquelle $R_3$ et $R_4$ ont les significations indiquées ci-dessus; et

(C) pour préparer des composés répondant aux formules (XIV), (XV) et (XVI) :

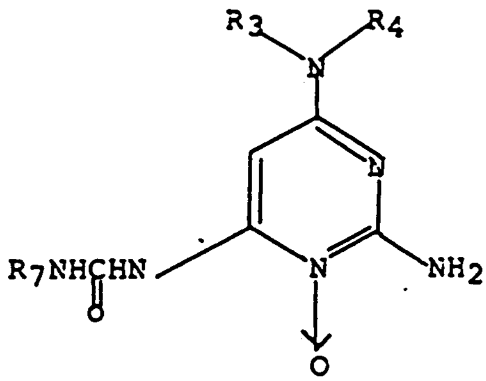

(XIV)

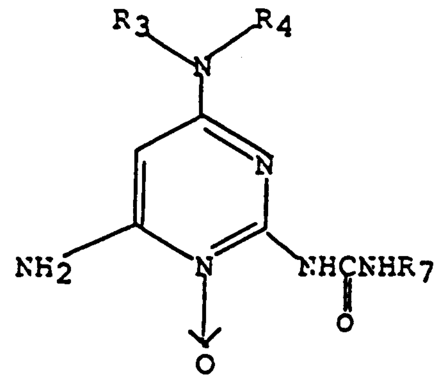

(XV)

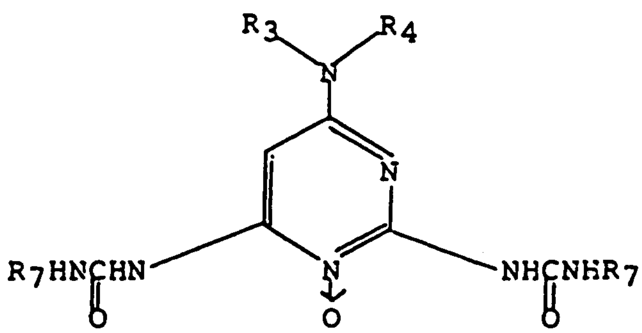

l'on traite un triamino-2,4,6 pyrimidine oxyde-3 par un isocyanate répondant à la formule $R_7$-N=C=O et dans laquelle le groupement amino en position 6 désigne le groupement :

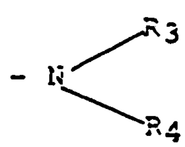

où $R_3$ et $R_4$ ont les significations indiquées ci-dessus, dans un solvant aprotique polaire à une température comprise entre 20 et 100 ° C.

**2.** Procédé selon la revendication 1(C), caractérisé par le fait que l'on fait réagir les mono-urées de formule (XIV) ou (XV) avec un isocyanate de formule $R_8$ - N = C = O :

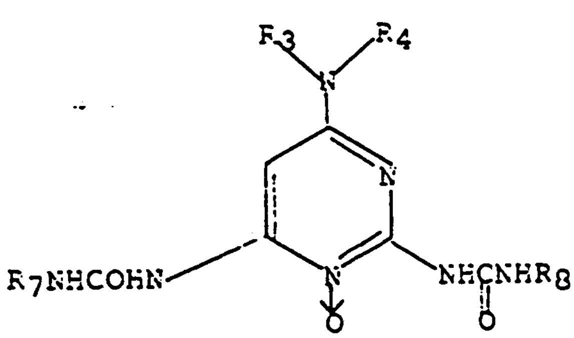

(XVII)

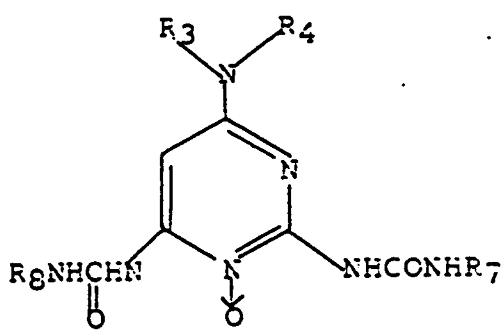

(XVIII)

dans lesquelles $R_3$, $R_4$ et $R_7$ ont les significations indiquées dans la revendication 1, et $R_8$, identique

ou différent de $R_7$, a les mêmes significations que $R_7$.

3. Procédé selon la revendication 1, caractérisé par le fait que dans la formule (I), le radical alkyle en $C_1$-$C_{18}$ est choisi parmi les radicaux méthyle, éthyle, propyle, éthyl-2 hexyle, octyle, dodécyle, hexadécyle et octadécyle, que le radical alkyle en $C_1$-$C_6$ est choisi parmi les groupements méthyle, éthyle, isopropyle, butyle et tertiobutyle, que les groupements alcényle en $C_2$-$C_{18}$ sont choisis parmi les groupements allyle, butényle, hexényle, dodécényle, hexadécényle et octadécényle, que les groupements aryle ou aralkyle sont choisis parmi las radicaux phényle, tolyl-4, nitro-2 phényle, nitro-4 phényle, fluoro-4 phényle, chloro-4 phényle, carboxy-2 phényle, carboxy-4 phényle, hydroxy-4 phényle, benzyle, phénéthyle; que l'atome d'halogène est un atome de chlore.

4. Procédé selon l'une quelconque des revendications 1 à 3, caractérisé par le fait que les groupements $R_3$ et $R_4$ forment avec l'atome d'azote auquel ils sont liés un groupement pipéridino et que $R_1$ et/ou $R_2$ désignent hydrogène, et $R_7$ et $R_8$ désignent un groupement alkyle inférieur, cycloalkyle en $C_5$-$C_8$, aryle ou aralkyle.

5. Composition se présentant sous forme d'onguents, de teintures, de crèmes, de pommades, de poudres, de timbres, de tampons imbibés, de solutions, d'émulsions, de lotions, de gels, de sprays ou de suspensions anhydres ou aqueuses, caractérisée par le fait qu'elle contient dans un support approprié pour une application topique, au moins un composé obtenu par le procédé dans l'une quelconque des revendications 1 à 4, dans des concentrations comprises entre 0,1 et 10% en poids par rapport au poids total de la composition, en particulier entre 0,2 et 5% en poids.

6. Composition cosmétique sous forme de lotions, de gels, de savons, de shampooings, caractérisée par le fait qu'elle contient dans un support physiologiquement acceptable, au moins l'un des composés tels qu'obtenus par le procédé dans l'une quelconque des revendications 1 à 4, à une concentration comprise entre 0,01 et 5% en poids.

7. Composition selon la revendication 6, caractérisée par le fait qu'elle contient en plus des agents hydratants, des agents antiséborrhéiques, des agents favorisant la repousse des cheveux, des agents anti-inflammatoires, stéroïdiens ou non stéroïdiens, des caroténoïdes, les acides eicosatétraynoïque-5,8,11,14 et eicosatriynoïque-5,8,11 et leurs esters et amides.

8. Composition selon l'une quelconque des revendications 5 à 8, caractérisée par le fait qu'elle contient également des agents conservateurs, des agents stabilisants, des agents régulateurs de pH, des agents modificateurs de pression osmotique, des agents émulsifiants, des filtres UV-A et UV-B, des agents antioxydants.

**Claims**

**Claims for the following Contracting States : AT, BE, CH, DE, FR, GB, IT, LI, NL, SE**

1. Compound characterised in that it is of the formula:

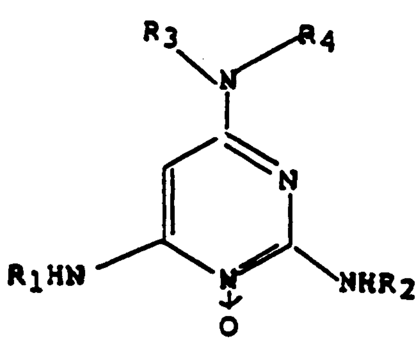

(I)

in which $R_3$ and $R_4$, identical or different, denote a hydrogen atom, a $C_1$-$C_{18}$ linear or branched alkyl radical, an alkenyl radical having 2 to 18 carbon atoms, a cycloalkyl radical of 5 to 8 carbon atoms,

optionally substituted by one or more $C_1$-$C_6$ alkyl groups, and the alkyl, alkenyl or cycloalkyl groups may themselves be substituted by one or more hydroxyl groups, $R_3$ and/or $R_4$ also denote an aryl or aralkyl group of the formula:

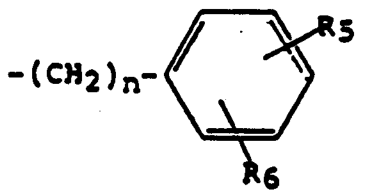

(II)

in which n may take values from 0 to 4 and where $R_5$ and/or $R_6$, independently of one another, denote hydrogen, a $C_1$-$C_6$ alkyl group, a nitro, hydroxyl or alkoxy group or a halogen atom or also a carboxylic group as well as the salified, ester and amide forms of the latter; $R_3$ and $R_4$ taken together with the nitrogen atom to which they are attached may form a morpholino, piperidino, pyrrolidino piperazino or 4'-N-alkylpiperazino group;
$R_1$ and/or $R_2$, independently of one another, denote a hydrogen or a carbamoyl group of formula:

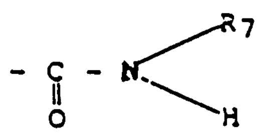

(III)

provided however that $R_1$ and $R_2$ do not denote simultaneously a hydrogen atom, and $R_7$ denotes a $C_1$-$C_{18}$ linear or branched alkyl radical, a $C_2$-$C_{18}$ alkenyl group, a $C_5$-$C_8$ cycloalkyl group, $R_7$ may also denote an aryl or aralkyl radical of the formula (II) defined above as well as their tautomeric form of the formulae:

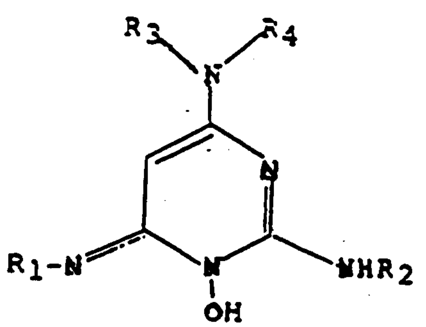

(IA)

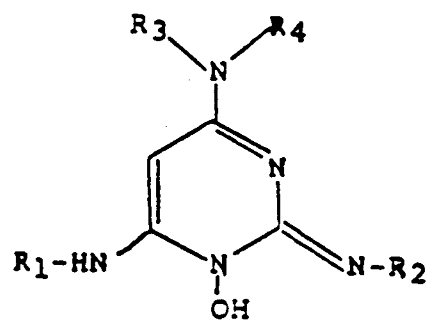

(IB)

2. Compound according to Claim 1, characterised in that in the formula (I), the $C_1$-$C_{18}$ alkyl radical is chosen from the methyl, ethyl, propyl, 2-ethylhexyl, octyl, dodecyl, hexadecyl and octadecyl radicals, in that the $C_1$-$C_6$ alkyl radical is chosen from the methyl, ethyl, isopropyl, butyl and tert-butyl groups, in that the $C_2$-$C_{18}$ alkenyl groups are chosen from the allyl, butenyl, hexenyl, dodecenyl, hexadecenyl and octadecenyl groups, in that the aryl or aralkyl groups are chosen from the phenyl, 4-tolyl, 2-nitrophenyl, 4-nitrophenyl, 4-fluorophenyl, 4-chlorophenyl, 2-carboxyphenyl, 4-carboxyphenyl, 4-hydroxyphenyl, benzyl and phenethyl radicals; and in that the halogen atom is a chlorine atom.

3. Compound according to Claim 1 or 2, characterised in that it is of the formula:

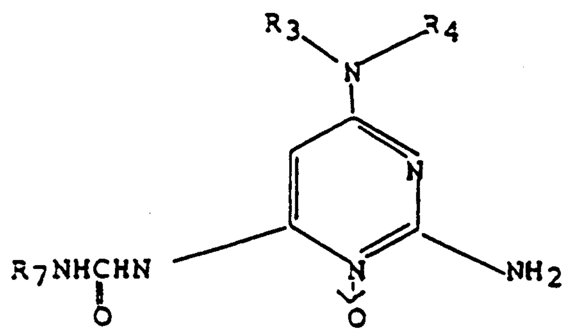

(XIV)

in which $R_3$, $R_4$ and $R_7$ have the meanings given in Claim 1.

**4.** Compound according to Claim 1 or 2, characterised in that it is of the formula:

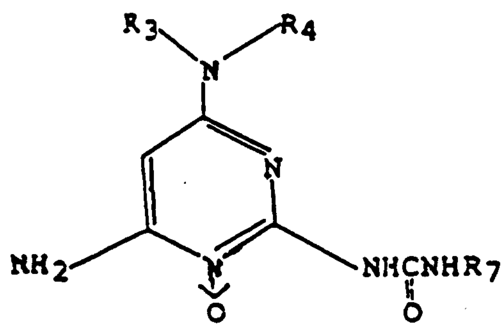

(XV)

in which $R_3$, $R_4$ and $R_7$ have the meanings given in Claim 1.

**5.** Compound according to Claim 1 or 2, characterised in that it is of the formula:

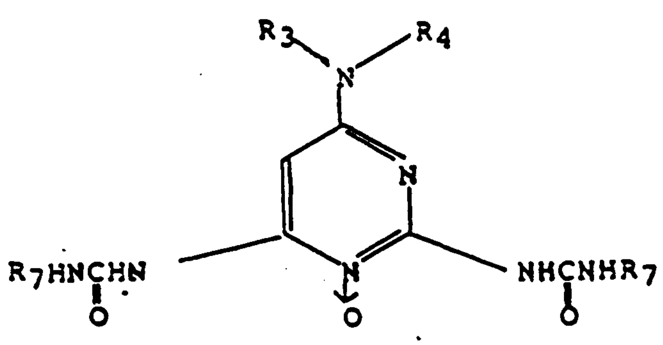

(XVI)

in which $R_3$, $R_4$ and $R_7$ have the meanings given in Claim 1.

**6.** Compound according to Claim 1 or 2, characterised in that the compound is of the formulae:

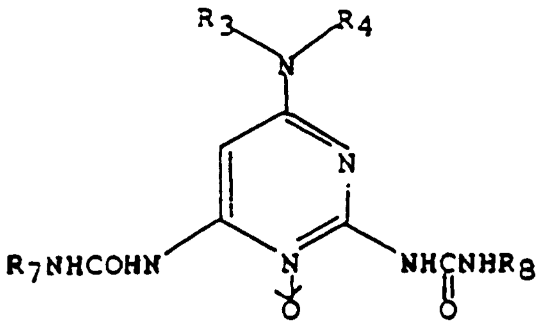

(XVII)

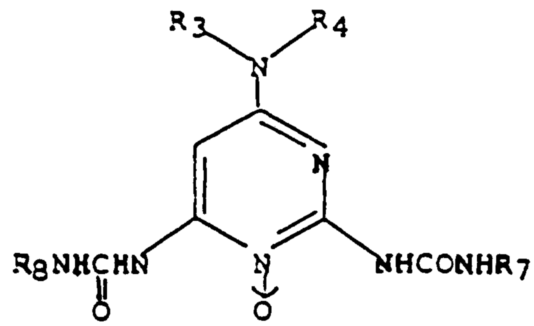

(XVIII)

in which $R_3$, $R_4$ and $R_7$ have the meanings given in Claim 1 and $R_8$ identical to or different from $R_7$, has the same meanings as $R_7$.

**7.** Compound according to any one of Claims 1 to 6, characterised in that the groups $R_3$ and $R_4$ form with the nitrogen atom to which they are attached a piperidino group and in that $R_1$ and/or $R_2$ denote hydrogen, and $R_7$ and $R_8$ denote a lower alkyl, a $C_5$-$C_8$ cycloalkyl, an aryl or an aralkyl group.

**8.** Process for the preparation of the compound according to Claim 1 or 2, in particular the compounds of the formula (VIII):

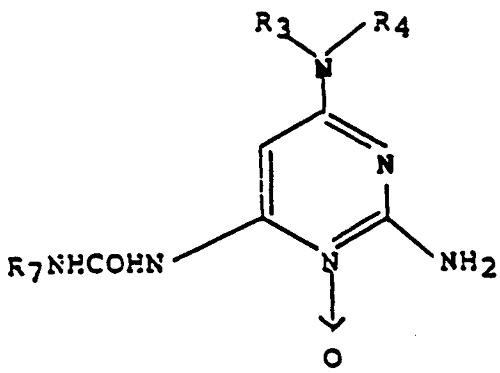

(VIII)

or (XIII)

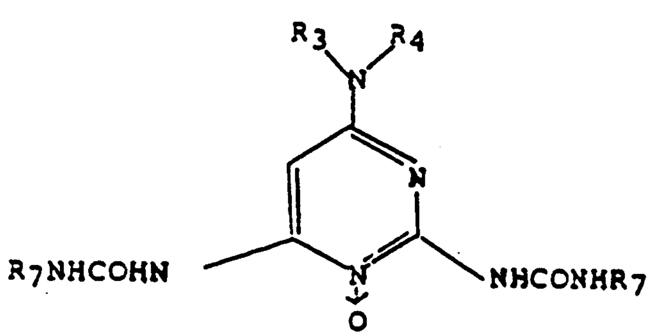

(XIII)

in which $R_3$, $R_4$ and $R_7$ have the same meanings as those given in Claim 1, characterised in that 6-chloro-2,4-diaminopyrimidine is treated with an isocyanate of formula $R_7$-N = C = O, in which $R_7$ has the meanings given in Claim 1, in a polar aprotic organic solvent, in that the oxidation of the derivatives resulting therefrom is then carried out and in that the procedure is followed by a treatment with an amine of the formula

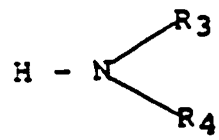

in which $R_3$ and $R_4$ have the meanings given in Claim 1.

**9.** Process for the preparation of the compounds according to Claim 1 or 2, in particular of the compounds of the formula:

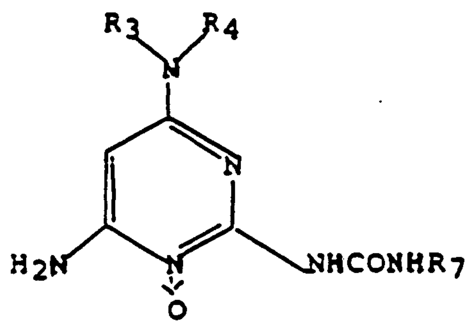

(XI)

in which $R_3$, $R_4$ and $R_7$ have the meanings given in Claim 1, by treating 6-chloro-2,4-diaminopyrimidine with an isocyanate of formula $R_7$-N=C=O in tetrahydrofuran, where $R_7$ has the meanings given in Claim 1. The product from the condensation of the isocyanate with 6-hydroxy-2,4-diaminopyrimidine obtained in N-methylpyrrolidone is then converted to the corresponding tosylate, the latter or the derivative from the condensation of the isocyanate with 6-chloro-2,4-diaminopyrimidine being oxidised to the corresponding N-oxides which are reacted with an amine of formula:

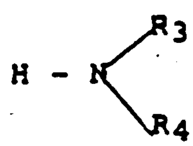

in which $R_3$ and $R_4$ have the meanings given above.

**10.** Process for the preparation of the compounds according to Claim 1 or 2, in particular the compounds of the formulae (XIV), (XV) and (XVI):

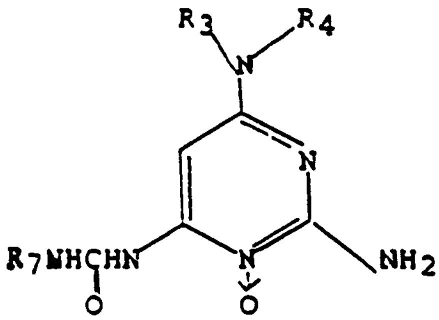

(XIV)

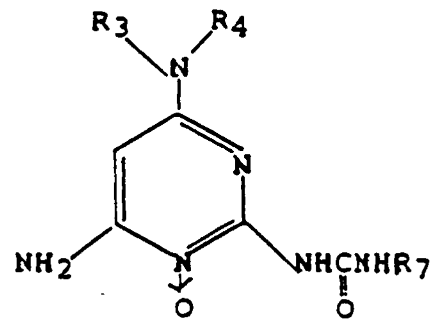

(XV)

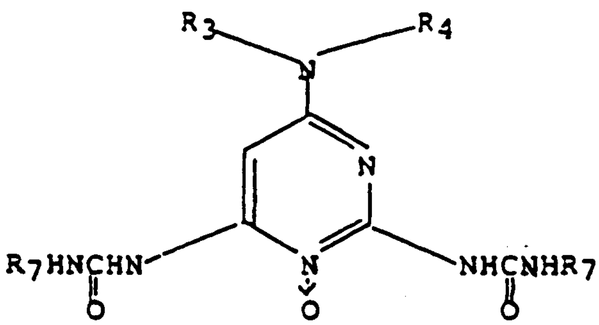

characterised in that a 2,4,6-triaminopyrimidine 3-oxide is treated with an isocyanate of the formula $R_7$-N=C=O, and in which the amino group in position 6 denotes the group

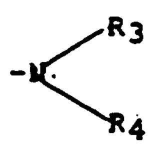

where $R_3$ and $R_4$ have the meanings given above, in a polar aprotic solvent at a temperature of between 20 and 100 ° C.

**11.** Process according to Claim 10, characterised in that the mono-ureas of formula (XIV) or (XV) are reacted with an isocyanate of formula $R_8$-N=C=O:

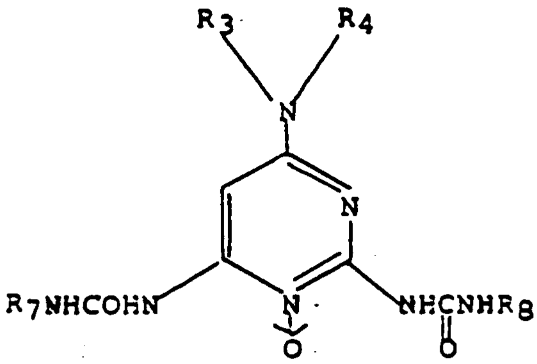

(XVII)

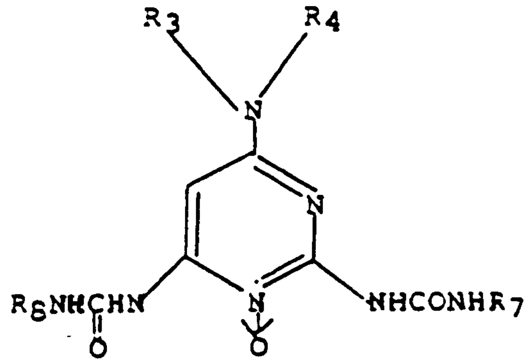

(XVIII)

in which $R_3$, $R_4$ and $R_7$ have the meanings given in Claim 1 and $R_8$, identical to or different from $R_7$, has the same meanings as $R_7$.

**12.** Cosmetic or pharmaceutical composition characterised in that it contains in a carrier suitable for a topical application at least one compound from any one of Claims 1 to 7.

**13.** Pharmaceutical composition according to Claim 12, characterised in that it is provided in the form of ointments, dyes, creams, pomades, powders, patches, impregnated pads, solutions, emulsions, lotions, gels, sprays or anhydrous or aqueous suspensions, containing at least one compound such as is defined in any one of Claims 1 to 7.

**14.** Composition according to Claim 13, characterised in that the compounds of formula (I) are provided in concentrations of between 0.1 and 10% by weight relative to the total weight of the composition, in particular between 0.2 and 5% by weight.

**15.** Cosmetic competition according to Claim 12 in the form of lotions, gels, soaps or shampoos, characterised in that it contains in a physiologically acceptable carrier at least one of the compounds such as are defined in any one of Claims 1 to 7 at a concentration of between 0.01 and 5% by weight.

**16.** Composition according to Claim 15, characterised in that it contains, in addition, moisturising agents, antiseborrhoeic agents, agents promoting the regrowth of hair, anti-inflammatory, steroidal or non-steroidal agents, carotenoids, 5,8,11,14-eicosatetraynoic and 5,8,11-eicosatriynoic acids and their esters and amides.

**17.** Composition according to any one of Claims 15 to 16, characterised in that it also contains preserving agents, stabilising agents, pH-regulating agents, osmotic pressure-modifying agents, emulsifying agents, UV-A and UV-B filters and antioxidising agents.

**18.** Process for the cosmetic treatment of hair, characterised in that at least one composition such as is defined in any one of Claims 14 to 17 is applied to the scalp.

**19.** Use of the compounds according to any one of Claims 1 to 7 for the preparation of a medicinal substance for the treatment of alopecia, loss of hair and desquamative dermatitis.

**Claims for the following Contracting State : ES**

**1.** Process for the preparation of the compound which is of the formula:

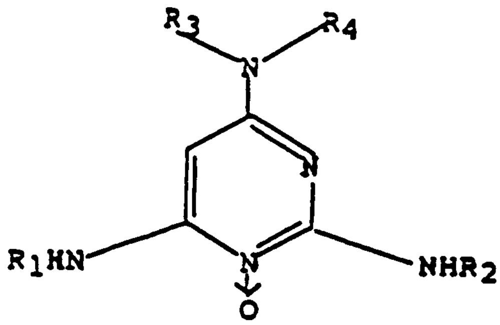

(I)

in which $R_3$ and $R_4$, identical or different, denote a hydrogen atom, a $C_1$-$C_{18}$ linear or branched alkyl radical, an alkenyl radical having 2 to 18 carbon atoms, a cycloalkyl radical of 5 to 8 carbon atoms, optionally substituted by one or more $C_1$-$C_6$ alkyl groups, and the alkyl, alkenyl or cycloalkyl groups may themselves be substituted by one or more hydroxyl groups, $R_3$ and/or $R_4$ also denote an aryl or aralkyl group of the formula:

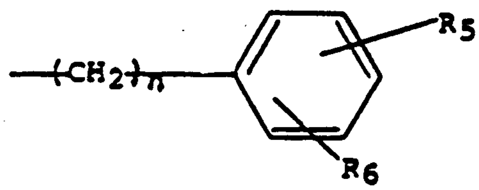

(II)

in which n may take values from 0 to 4 and where $R_5$ and/or $R_6$, independently of one another, denote hydrogen, a $C_1$-$C_6$ alkyl group, a nitro, hydroxyl or alkoxy group or a halogen atom or also a carboxylic group as well as the salified, ester and amide forms of the latter; $R_3$ and $R_4$ taken together with the nitrogen atom to which they are attached may form a morpholino, piperidino, pyrrolidino, piperazino or 4'-N-alkylpiperazino group;

$R_1$ and/or $R_2$, independently of one another, denote a hydrogen or a carbamoyl group of formula:

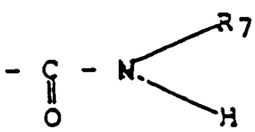

(III)

provided however that $R_1$ and $R_2$ do not denote simultaneously a hydrogen atom, and $R_7$ denotes a $C_1$-$C_{18}$ linear or branched alkyl radical, a $C_2$-$C_{18}$ alkenyl group, a $C_5$-$C_8$ cycloalkyl group, $R_7$ may also denote an aryl or aralkyl radical of the formula (II) defined above as well as their tautomeric form of the formulae:

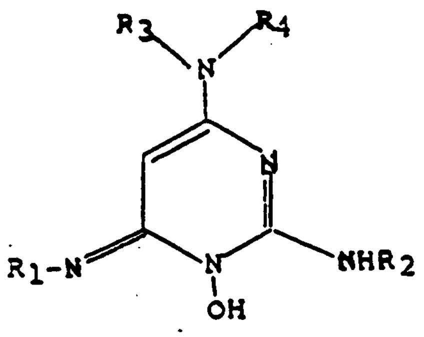

(IA)

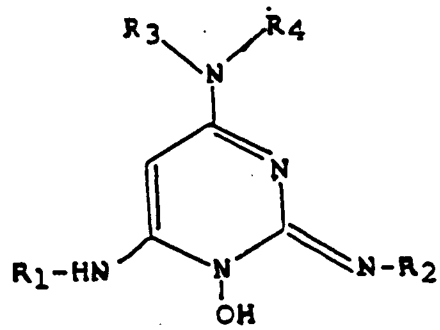

(IB)

characterised in that:

(A) in order to prepare compounds of the formula (VIII):

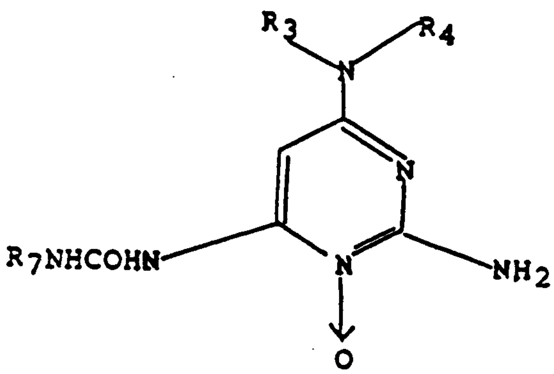

(VIII)

or (XIII)

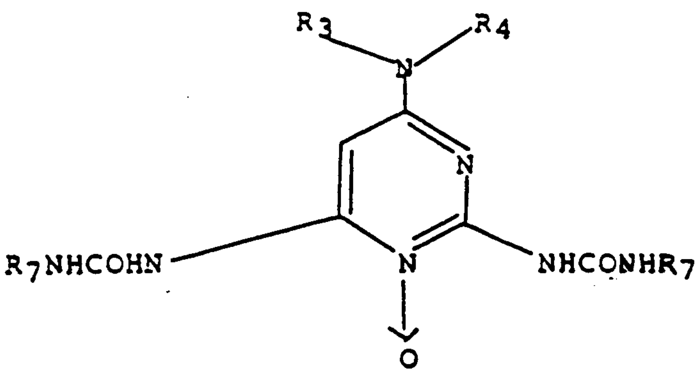

(XIII)

in which $R_3$, $R_4$ and $R_7$ have the same meanings as those given above, 6-chloro-2,4-diaminopyrimidine is treated with an isocyanate of formula $R_7\text{-}N=C=O$, in which $R_7$ has the meanings given above, in a polar aprotic organic solvent, in that the oxidation of the derivatives resulting therefrom is then carried out and in that the procedure is followed by a treatment with an amine of the formula

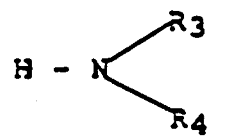

in which $R_3$ and $R_4$ have the meanings given above;
(B) in order to prepare compounds of the formula:

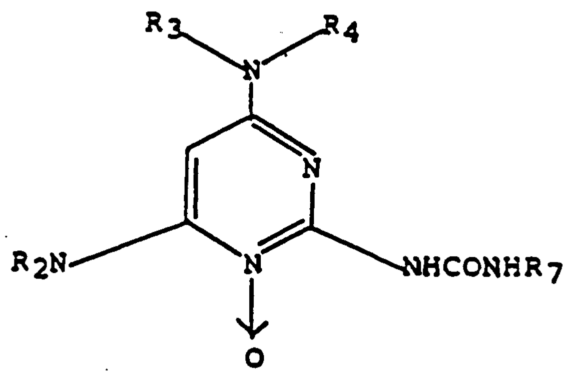

(XI)

in which $R_3$, $R_4$ and $R_7$ have the meanings given above, 6-chloro-2,4-diaminopyrimidine is treated with an isocyanate of formula $R_7$-N=C=O in tetrahydrofuran, where $R_7$ have the meanings given above 1. The product from the condensation of the isocyanate with 6-hydroxy-2,4-diaminopyrimidine obtained in N-methylpyrrolidone is then converted to the corresponding tosylate, the latter or the derivative from the condensation of the isocyanate with 6-chloro-2,4-diaminopyrimidine being oxidised to the corresponding N-oxides which are reacted with an amine of formula:

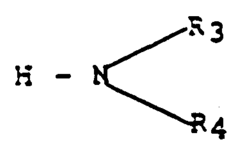

in which $R_3$ and $R_4$ have the meanings given above; and
(C) in order to prepare compounds of the formulae (XIV), (XV) and (XVI):

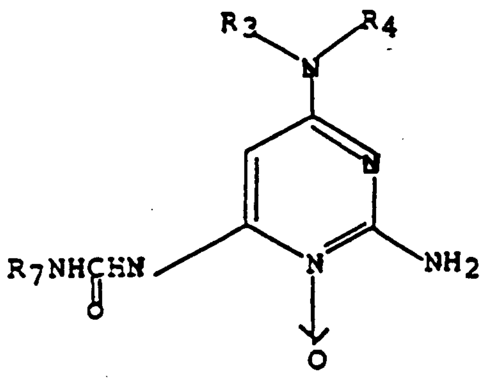

(XIV)

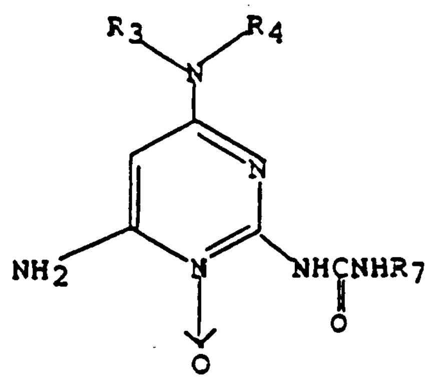

(XV)

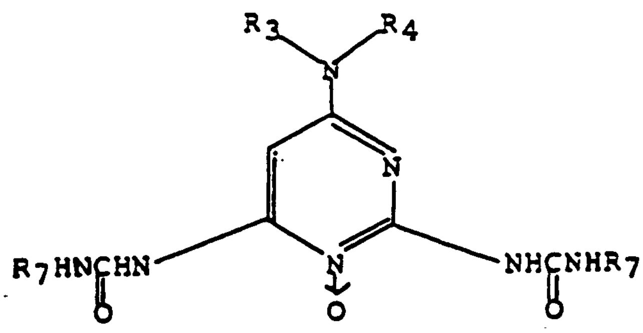

a 2,4,6-triaminopyrimidine 3-oxide is treated with an isocyanate of the formula $R_7$-N = C = O, and in which the amino group in position 6 denotes the group:

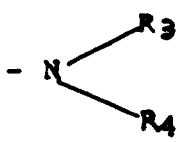

where $R_3$ and $R_4$ have the meanings given above, in a polar aprotic solvent at a temperature of between 20 and 100 ° C.

**2.** Process according to Claim 1(C), characterised in that the mono-ureas of formula (XIV) or (XV) are reacted with an isocyanate of formula $R_8$-N = C = O:

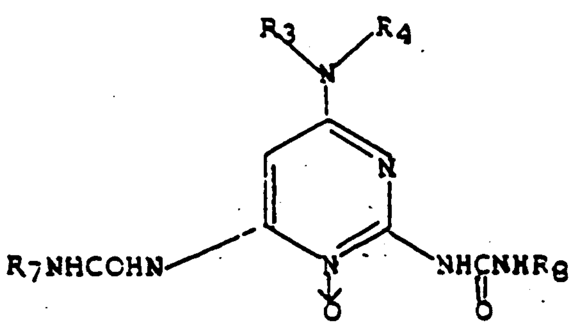

(XVII)

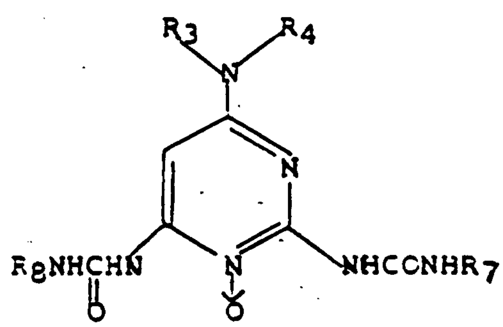

(XVIII)

in which $R_3$, $R_4$ and $R_7$ have the meanings given in Claim 1 and $R_8$, identical to or different from $R_7$, has the same meanings as $R_7$.

3. Process according to Claim 1, characterised in that in the formula (I), the $C_1$-$C_{18}$ alkyl radical is chosen from the methyl, ethyl, propyl, 2-ethylhexyl, octyl, dodecyl, hexadecyl and octadecyl radicals, in that the $C_1$-$C_6$ alkyl radical is chosen from the methyl, ethyl, isopropyl, butyl and tert-butyl groups, in that the $C_2$-$C_{18}$ alkenyl groups are chosen from the allyl, butenyl, hexenyl, dodecenyl, hexadecenyl and octadecenyl groups, in that the aryl or aralkyl groups are chosen from the phenyl, 4-tolyl, 2-nitrophenyl, 4-nitrophenyl, 4-fluorophenyl, 4-chlorophenyl, 2-carboxyphenyl, 4-carboxyphenyl, 4-hydroxyphenyl, benzyl and phenethyl radicals; in that the halogen atom is a chlorine atom.

4. Process according to any one of Claims 1 to 3, characterised in that the groups $R_3$ and $R_4$ form with the nitrogen atom to which they are attached a piperidino group and in that $R_1$ and/or $R_2$ denote hydrogen, and $R_7$ and $R_8$ denote a lower alkyl, a $C_5$-$C_8$ cycloalkyl, an aryl or an aralkyl group.

5. Composition provided in the form of ointments, dyes, creams, pomades, powders, patches, impregnated pads, solutions, emulsions, lotions, gels, sprays or anhydrous or aqueous suspensions, characterised in that it contains in a carrier suitable for a topical application, at least one compound obtained by the process in any one of Claims 1 to 4, in concentrations of between 0.1 and 10% by weight relative to the total weight of the composition, in particular between 0.2 and 5% by weight.

6. Cosmetic composition in the form of lotions, gels, soaps or shampoos, characterized in that it contains in a physiologically acceptable carrier at least one of the compounds such as are obtained by the process in any one of Claims 1 to 4 at a concentration of between 0.01 and 5% by weight.

7. Composition according to Claim 6, characterised in that it contains, in addition, moisturizing agents, antiseborrhoeic agents, agents promoting the regrowth of hair, anti-inflammatory, steroidal or non-steroidal agents, carotenoids, 5,8,11,14-eicosatetraynoic and 5,8,11-eicosatriynoic acids and their esters and amides.

8. Composition according to any one of Claims 5 to 8, characterised in that it also contains preserving agents, stabilising agents, pH-regulating agents, osmotic pressure-modifying agents, emulsifying agents, UV-A and UV-B filters and antioxidising agents.

**Patentansprüche**
**Patentansprüche für folgende Vertragsstaaten : AT, BE, CH, DE, FR, GB, IT, LI, NL, SE**

1. Verbindung der Formel:

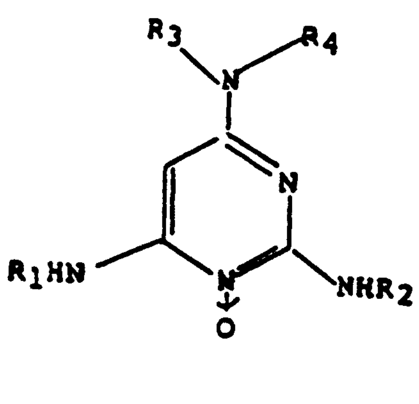

(I)

worin $R_3$ und $R_4$, gleich oder verschieden, Wasserstoff, einen linearen oder verzweigten $C_1$-$C_{18}$-Alkylrest, eine Alkenylgruppe mit 2 bis 18 Kohlenstoffatomen, eine Cycloalkylgruppe mit 5 bis 8 Kohlenstoffatomen darstellen, welche gegebenenfalls durch eine oder mehrere $C_1$-$C_6$-Alkylgruppe(n) substituiert sind, wobei die Alkyl-, Alkenyl- oder Cycloalkylgruppen ihrerseits durch eine oder mehrere Hydroxylgruppe(n) substituiert sein können, $R_3$ und/oder $R_4$ auch eine Aryl- oder Aralkylgruppe der Formel darstellen:

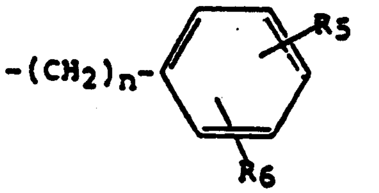

(II)

worin n die Werte 0 bis 4 annehmen kann und worin $R_5$ und/oder $R_6$, unabhängig voneinander, Wasserstoff, eine $C_1$-$C_6$ Alkylgruppe, eine Nitro-, Hydroxyl-, Alkoxygruppe oder ein Halogenatom oder auch eine Carboxylgruppe sowie die Salz-, Ester- und Amidformen der letzteren darstellen; $R_3$ und $R_4$ zusammen mit dem Stickstoffatom, an das sie gebunden sind, eine Morpholin-, Piperidin-, Pyrrolidin-, Piperazin- und N-Alkyl-4'-piperazingruppe bilden können;
$R_1$ und/oder $R_2$, unabhängig voneinander, Wasserstoff oder eine Carbamoylgruppe der Formel darstellen:

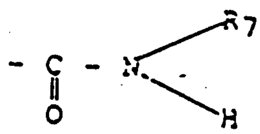

(III)

mit der Maßgabe, daß jedesmal wenn $R_1$ und $R_2$ nicht gleichzeitig Wasserstoff bedeuten und $R_7$ einen linearen oder verzweigten $C_1$-$C_{18}$-Alkylrest, eine $C_2$-$C_{18}$-Alkenylgruppe, eine $C_5$-$C_8$-Cycloalkylgruppe darstellt, $R_7$ auch einen Aryl- oder Aralkylrest der oben definierten Formel (II) darstellen kann,
sowie deren tautomere Form der Formeln:

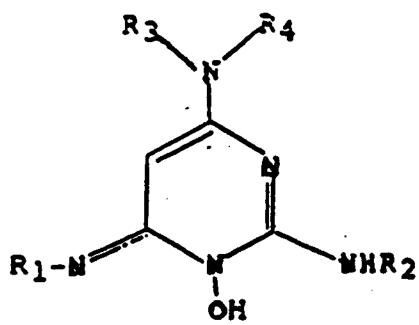

(IA)

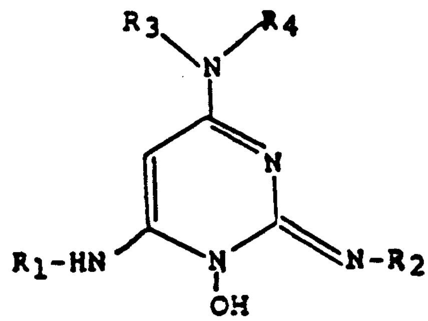

(IB)

2. Verbindung gemäß Anspruch 1,
dadurch **gekennzeichnet,** daß
in der Formel (I) der $C_1$-$C_{16}$-Alkylrest unter Methyl-, Ethyl-, Propyl-, Ethyl-2-hexyl-, Octyl-, Dodecyl-, Hexadecyl- und Octadecylresten, der $C_1$-$C_6$-Alkylrest unter Methyl-, Ethyl-, Isopropyl-, Butyl- und t-Butylgruppen, die $C_2$-$C_{18}$-Alkenylgruppe unter Alkyl-, Butenyl-, Hexenyl-, Dodecenyl-, Hexadecenyl- oder Octadecenylgruppen, die Aryl- oder Aralkylgruppe unter Phenyl-, Tolyl-4-, Nitro-2-phenyl-, Nitro-4-phenyl-, Fluor-4-phenyl-, Chlor-4-phenyl-, Carboxy-2-phenyl-, Carboxy-4-phenyl-, Hydroxy-4-phenyl-, Benzyl-, Phenethylresten ausgewählt sind und daß das Halogenatom ein Chloratom ist.

3. Verbindung gemäß Anspruch 1 oder 2,
dadurch **gekennzeichnet,** daß
sie die Formel aufweist:

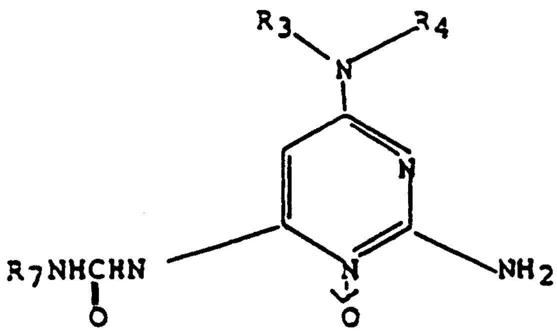

(XIV)

worin $R_3$, $R_4$ und $R_7$ die in Anspruch 1 angegebene Bedeutung haben.

**4.** Verbindung gemäß Anspruch 1 oder 2, dadurch **gekennzeichnet,** daß sie die Formel aufweist:

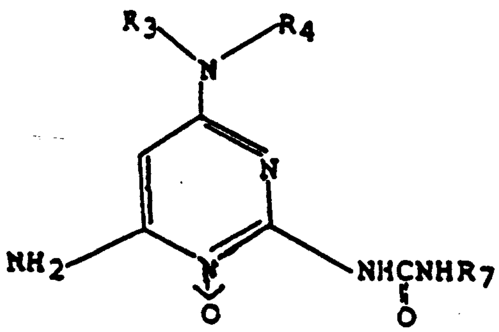

(XV)

worin $R_3$, $R_4$ und $R_7$ die in Anspruch 1 angegebene Bedeutung haben.

**5.** Verbindung gemäß Anspruch 1 oder 2, dadurch **gekennzeichnet,** daß sie die Formel aufweist:

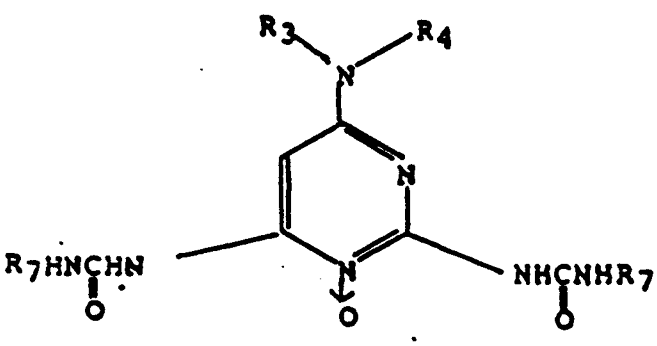

(XVI)

worin $R_3$, $R_4$ und $R_7$ die in Anspruch 1 angegebene Bedeutung haben.

**6.** Verbindung gemäß Anspruch 1 oder 2, dadurch **gekennzeichnet,** daß sie die Formeln aufweist:

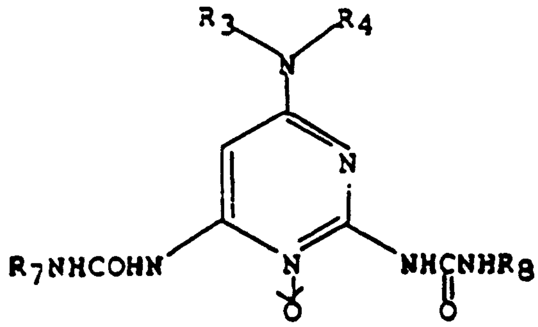

(XVII)

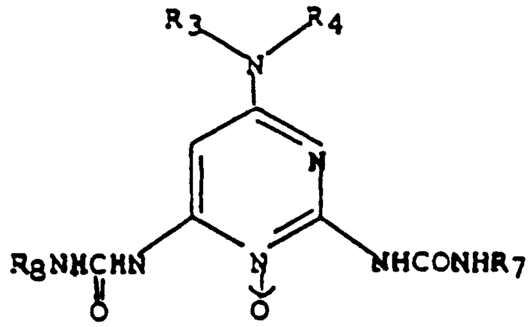

(XVIII)

worin $R_3$, $R_4$ und $R_7$ die in Anspruch 1 angegebene Bedeutung und $R_8$, gleich oder verschieden (mit) von $R_7$, die gleichen Bedeutungen wie $R_7$ haben.

**7.** Verbindung eines jeden der Ansprüche 1 bis 6,
dadurch **gekennzeichnet,** daß
die Gruppen $R_3$ und $R_4$ mit dem Stickstoffatom, an das sie gebunden sind, eine Piperidingruppe bilden und daß $R_1$ und/oder $R_2$ Wasserstoff und $R_7$ und $R_8$ eine Niedrigalkyl-, $C_5$-$C_8$-Cycloalkyl-, Aryl- oder Aralkylgruppe darstellen.

**8.** Verfahren zur Herstellung der Verbindung gemäß Anspruch 1 oder 2, insbesondere von Verbindungen der Formel (VIII):

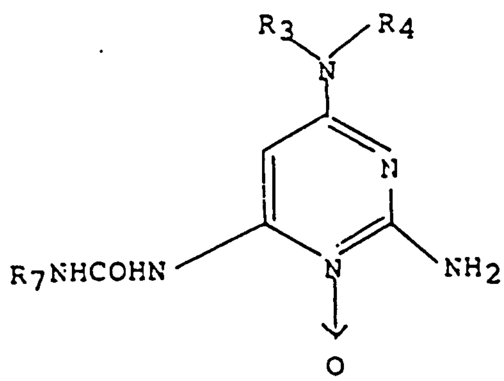

(VIII)

oder der Formel (XIII):

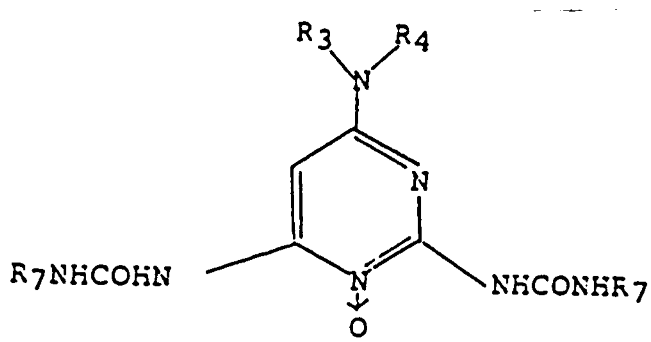

(XIII)

worin $R_3$, $R_4$ und $R_7$ dieselben Bedeutungen wie die in Anspruch 1 angegebenen haben,
dadurch **gekennzeichnet,** daß
man Chlor-6-diamino-2,4-pyrimidin mit einem Isocyanat der Formel $R_7$-N=C=O, worin $R_7$ die in

Anspruch 1 angegebenen Bedeutungen hat, in einem aprotischen polaren organischen Lösungsmittel behandelt, danach die dabei entstehenden Derivate oxidiert und anschließend mit einem Amin der Formel

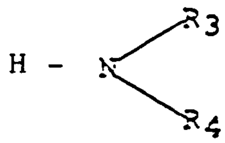

behandelt, worin $R_3$ und $R_4$ die in Anspruch 1 angegebenen Bedeutungen haben.

**9.** Verfahren zur Herstellung von Verbindungen gemäß des Anspruchs 1 oder 2, insbesondere von Verbindungen der Formel :

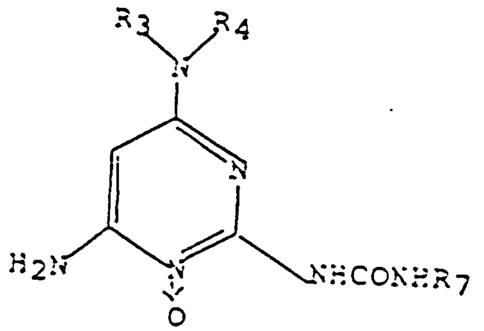

(XI)

worin $R_3$, $R_4$ und $R_7$ die in Anspruch 1 angegebenen Bedeutungen haben, wobei man Chlor-6-diamino-2,4-pyrimidin mit einem Isocyanat der Formel $R_7$-N=C=O, worin $R_7$ die in Anspruch 1 angegebenen Bedeutungen hat, in Tetrahydrofuran behandelt oder das in N-Methylpyrrolidon erhaltene Kondensationsprodukt des Isocyanats mit Hydroxy-6-diamino-2,4-pyrimidin sodann in das entsprechende Tosylat überführt, worauf das letztere oder das Kondensationsderivat des Isocyanats mit Chlor-6-diamino-2,4-pyrimidin, welche zu den entsprechenden N-Oxiden oxidiert werden, mit einem Amin der Formel reagieren läßt:

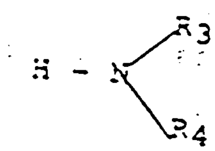

worin $R_3$ und $R_4$ die oben angegebenen Bedeutungen haben.

**10.** Verfahren zur Herstellung der Verbindungen gemäß Anspruch 1 oder 2, insbesondere der Verbindungen gemäß der Formeln (XIV), (XV) und (XVI):

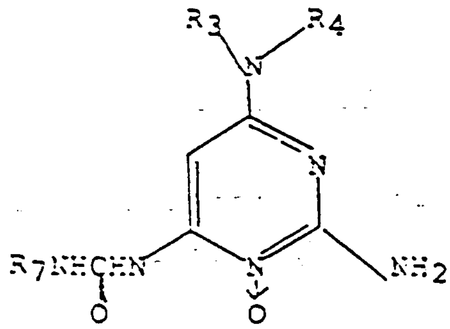

(XIV)

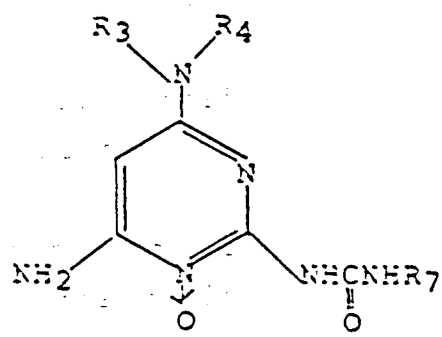

(XV)

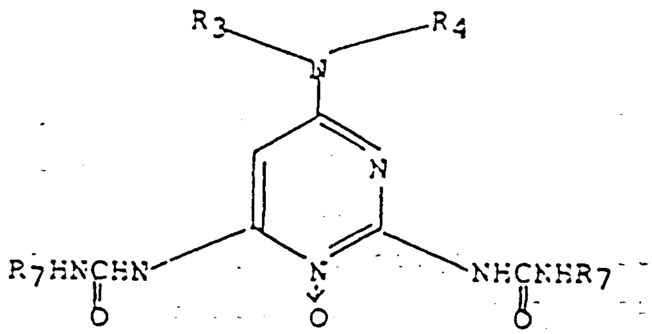

(XVI)

dadurch **gekennzeichnet,** daß
man ein Triamino-2,4,6-pyrimidin-oxid-3, worin die Aminogruppe in 6-Stellung die

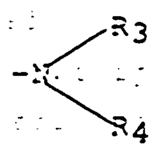

-Gruppe

darstellt, worin $R_3$ und $R_4$ die oben angegebenen Bedeutungen haben, mit einem Isocyanat der Formel $R_7$-N=C=O in einem aprotischen polaren Lösungsmittel bei einer Temperatur zwischen 20 und 100 °C behandelt.

**11.** Verfahren gemäß Anspruch 10,
dadurch **gekennzeichnet,** daß
man die Mono-Harnstoffverbindungen der Formel (XIV) oder (XV) mit einem Isocyanat der Formel $R_8$-N=C=O reagieren läßt zu:

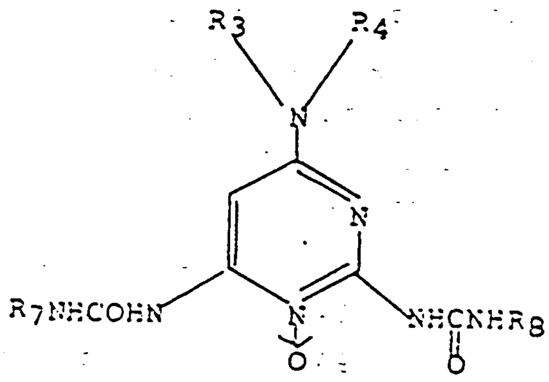

(XVII)

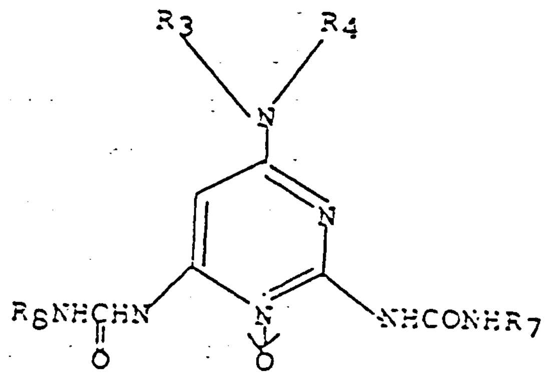

(XVIII)

worin $R_3$, $R_4$ und $R_7$ die in Anspruch 1 angegebenen Bedeutungen und $R_8$, gleich oder verschieden (mit) von $R_7$, die gleichen Bedeutungen wie $R_7$ haben.

**12.** Kosmetisches oder pharmazeutisches Mittel,
dadurch **gekennzeichnet,** daß
es in einem zur topischen Anwendung geeigneten Träger mindestens eine Verbindung gemäß eines jeden der Ansprüche 1 bis 7 enthält.

**13.** Pharmazeutisches Mittel gemäß des Anspruchs 12,
dadurch **gekennzeichnet,** daß
es in Form von Salben, Tinkturen, Crèmes, Pomaden, Pulvern, Tupfern, getränkten Tampons, Lösungen, Emulsionen, Lotionen, Gelen, Sprays oder wasserfreien oder wässrigen Suspensionen vorliegt, welche mindestens eine Verbindung gemäß eines jeden der Ansprüche 1 bis 7 enthalten.

**14.** Mittel gemäß Anspruch 13,
dadurch **gekennzeichnet,** daß
die Verbindungen der Formel (I) in Konzentrationen von 0,1 bis 10 Gew.%, bezogen auf Gesamtgewicht des Mittels, insbesondere von 0,2 bis 5 Gew.% vorliegen.

**15.** Kosmetisches Mittel gemäß Anspruch 12 in Form von Lotionen, Gelen, Seifen, Shampoos,
dadurch **gekennzeichnet,** daß
es in einem physiologisch verträglichen Träger mindestens eine der Verbindungen gemäß eines jeden der Ansprüche 1 bis 7 in einer Konzentration von 0,01 bis 5 Gew.% enthält.

**16.** Mittel gemäß Anspruch 15,
dadurch **gekennzeichnet,** daß
es zusätzlich hydratisierende Mittel, antiseborrheische Mittel, Mittel zur Förderung des Haarwuchses, steroidische oder nicht-steroidische Anti-Entzündungsmittel, Carotinoide, Eicosantetrain-5,8,11,14- und Eicosantriin-5,8,11-Säuren sowie deren Ester und Amide enthält.

**17.** Mittel gemäß eines jeden der Ansprüche 15 bis 16,
dadurch **gekennzeichnet,** daß
es auch Konservierungs-, Stabilisierungs-, pH-Regulierungs-, Modifikationsmittel des osmotischen Drukkes, Emulgatoren, UV-A- und UV-B-Filter, Antioxidantien enthält.

**18.** Verfahren zur kosmetischen Behandlung der Haare,
dadurch **gekennzeichnet,** daß
man auf behaarte Haut mindestens ein Mittel gemäß eines jeden der Ansprüche 14 bis 17 aufbringt.

**19.** Verwendung der Verbindungen gemäß eines jeden der Ansprüche 1 bis 7 zur Herstellung eines Medikaments zur Behandlung von Haarschwund, Haarausfall und schuppender Dermatitis.

**Patentansprüche für folgenden Vertragsstaat : ES**

1. Verfahren zur Herstellung einer Verbindung der Formel:

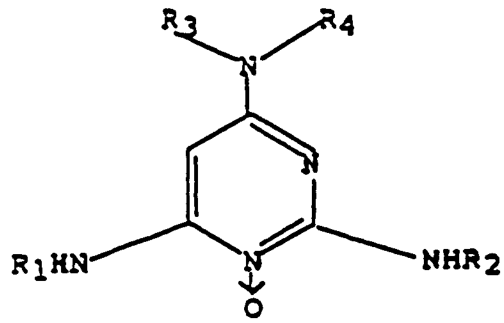

(I)

worin $R_3$ und $R_4$, gleich oder verschieden, Wasserstoff, einen linearen oder verzweigten $C_1$-$C_{18}$-Alkylrest, eine Alkenylgruppe mit 2 bis 18 Kohlenstoffatomen, eine Cycloalkylgruppe mit 5 bis 8 Kohlenstoffatomen darstellen, welche gegebenenfalls durch eine oder mehrere $C_1$-$C_6$-Alkylgruppe(n) substituiert sind, wobei die Alkyl-, Alkenyl- oder Cycloalkylgruppen ihrerseits durch eine oder mehrere Hydroxylgruppe(n) substituiert sein können, $R_3$ und/oder $R_4$ auch eine Aryl- oder Aralkylgruppe der Formel darstellen:

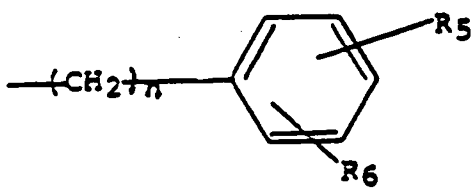

(II)

worin n die Werte 0 bis 4 annehmen kann und worin $R_5$ und/oder $R_6$, unabhängig voneinander, Wasserstoff, eine $C_1$-$C_6$ Alkylgruppe, eine Nitro-, Hydroxyl-, Alkoxygruppe oder ein Halogenatom oder auch eine Carboxylgruppe sowie die Salz-, Ester- und Amidformen der letzteren darstellen; $R_3$ und $R_4$ zusammen mit dem Stickstoffatom, an das sie gebunden sind, eine Morpholin-, Piperidin-, Pyrrolidin, Piperazin- und N-Alkyl-4'-piperazingruppe bilden können;
$R_1$ und/oder $R_2$, unabhängig voneinander, Wasserstoff oder eine Carbamoylgruppe der Formel darstellen:

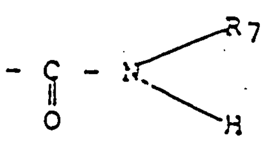

(III)

mit der Maßgabe, daß jedesmal wenn $R_1$ und $R_2$ nicht gleichzeitig Wasserstoff bedeuten und $R_7$ einen linearen oder verzweigten $C_1$-$C_{18}$-Alkylrest, eine $C_2$-$C_{18}$-Alkenylgruppe, eine $C_5$-$C_8$-Cycloalkylgruppe darstellt, $R_7$ auch einen Aryl- oder Aralkylrest der oben definierten Formel (II) darstellen kann,
sowie deren tautomere Form der Formeln:

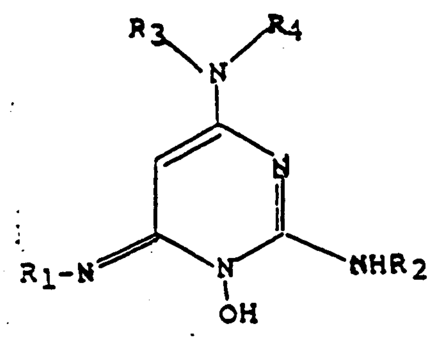

(IA)

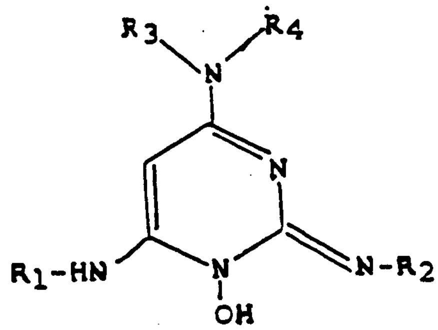

(IB)

dadurch **gekennzeichnet,** daß man:

(A) zur Herstellung der Verbindungen der Formel (VIII):

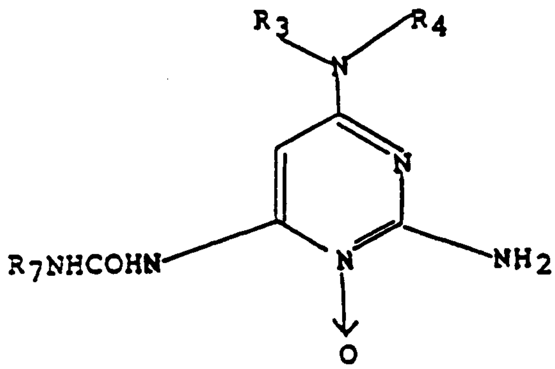

(VIII)

oder der Formel (XIII)

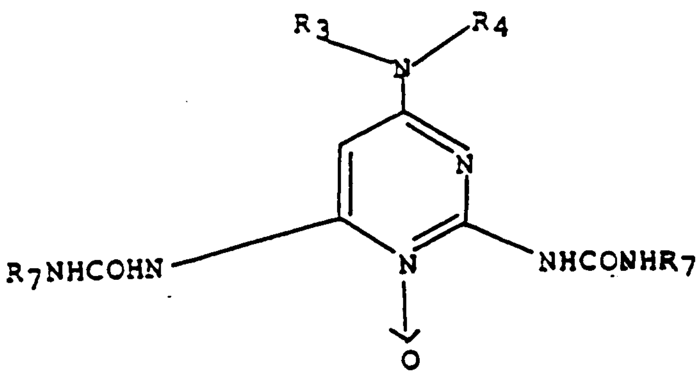

(XIII)

worin $R_3$, $R_4$ und $R_7$ dieselben Bedeutungen wie die in Anspruch 1 angegebenen haben, Chlor-6-diamino-2,4-pyrimidin mit einem Isocyanat der Formel $R_7\text{-}N=C=O$, worin $R_7$ die in Anspruch 1 angegebenen Bedeutungen hat, in einem aprotischen polaren organischen Lösungsmittel behandelt, danach die dabei entstehenden Derivate oxidiert und anschließend mit einem Amin der Formel

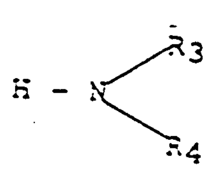

behandelt, worin $R_3$ und $R_4$ die in Anspruch 1 angegebenen Bedeutungen haben;

(B) zur Herstellung von Verbindungen der Formel :

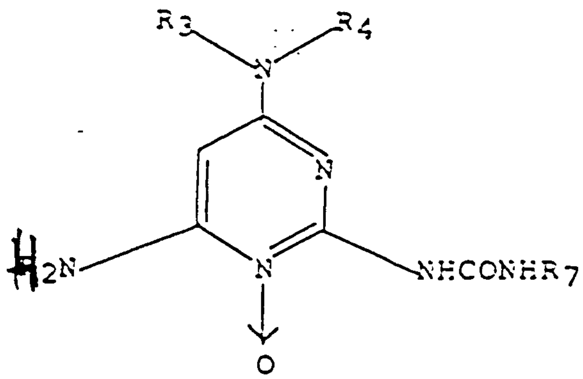

(XI)

worin $R_3$, $R_4$ und $R_7$ die in Anspruch 1 angegebenen Bedeutungen haben, Chlor-6-diamino-2,4-pyrimidin mit einem Isocyanat der Formel $R_7$-N=C=O, worin $R_7$ die in Anspruch 1 angegebenen Bedeutungen hat, in Tetrahydrofuran behandelt oder das in N-Methylpyrrolidon erhaltene Kondensationsprodukt des Isocyanats mit Hydroxy-6-diamino-2,4-pyrimidin sodann in das entsprechende Tosylat überführt, worauf das letztere oder das Kondensationsderivat des Isocyanats mit Chlor-6-diamino-2,4-pyrimidin, welche zu den entsprechenden N-Oxiden oxidiert werden, mit einem Amin der Formel reagieren läßt:

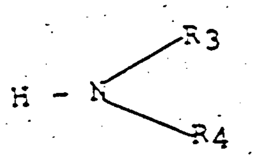

worin $R_3$ und $R_4$ die oben angegebenen Bedeutungen haben; und
(C) zur Herstellung der Verbindungen der Formeln (XIV), (XV) und (XVI):

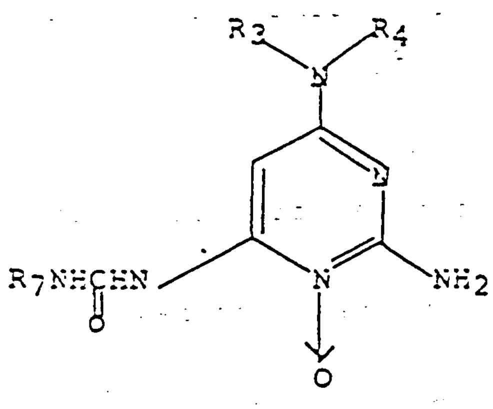

(XIV)

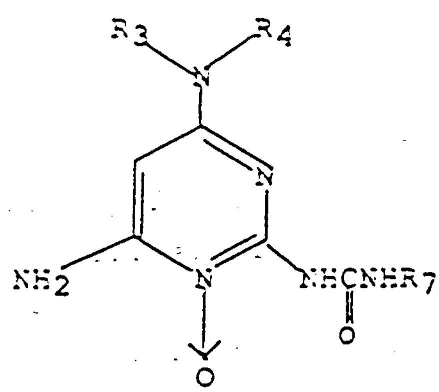

(XV)

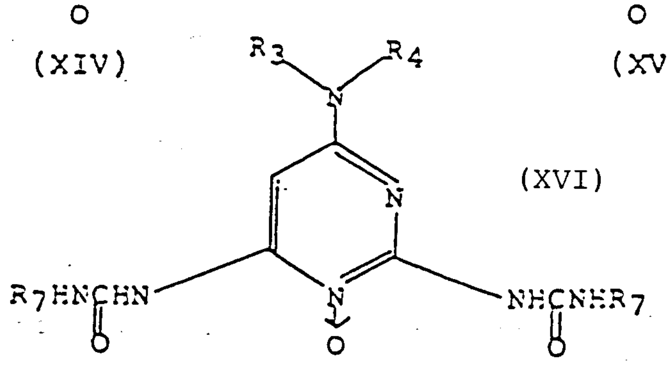

(XVI)

ein Triamino-2,4,6-pyrimidin-oxid-3, worin die Aminogruppe in 6-stellung die

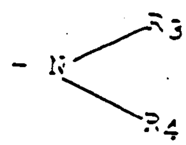

-Gruppe

darstellt, worin $R_3$ und $R_4$ die oben angegebenen Bedeutungen haben, mit einem Isocyanat der Formel $R_7$-N=C=O in einem aprotischen polaren Lösungsmittel bei einer Temperatur zwischen 20 und 100 °C behandelt.

2. Verfahren gemäß Anspruch 1 (C), dadurch **gekennzeichnet,** daß man die Mono-Harnstoffverbindungen der Formel (XIV) oder (XV) mit einem Isocyanat der Formel $R_8$-N=C=O reagieren läßt zu:

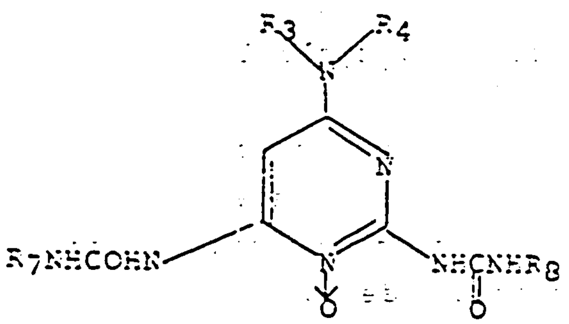

(XVII)

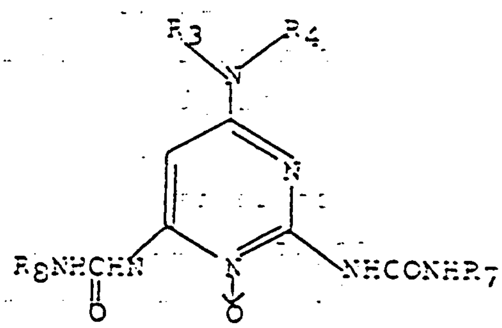

(XVIII)

worin $R_3$, $R_4$ und $R_7$ die in Anspruch 1 angegebenen Bedeutungen und $R_8$, gleich oder verschieden (mit) von $R_7$, die gleichen Bedeutungen wie $R_7$ haben.

3. Verfahren gemäß Anspruch 1, dadurch **gekennzeichnet,** daß in der Formel(I) der $C_1$-$C_{16}$ Alkylrest unter Methyl-, Ethyl-, Propyl-, Ethyl-2-hexyl-, Octyl-, Dodecyl-, Hexadecyl- und Octadecylresten, der $C_1$-$C_6$-Alkylrest unter Methyl-, Ethyl-, Isopropyl-, Butyl- und t-Butylgruppen, die $C_2$-$C_{18}$-Alkenylgruppe unter Alkyl-, Butenyl-, Hexenyl-, Dodecenyl-, Hexadecenyl- oder Octadecenylgruppen, die Aryl- oder Aralkylgruppe unter Phenyl-, Tolyl-4-, Nitro-2-phenyl-, Nitro-4-phenyl-, Fluor-4-phenyl-, Chlor-4-phenyl-, Carboxy-2-phenyl-, Carboxy-4-phenyl-, Hydroxy-4-phenyl-, Benzyl-, Phenethylresten ausgewählt sind und daß das Halogenatom ein Chloratom ist.

4. Verfahren gemäß eines jeden der Ansprüche 1 bis 3, dadurch **gekennzeichnet,** daß die Gruppen $R_3$ und $R_4$ mit dem Stickstoffatom, an das sie gebunden sind, eine Piperidingruppe bilden und daß $R_1$ und/oder $R_2$ Wasserstoff und $R_7$ und $R_8$ eine Niedrigalkyl-, $C_5$-$C_8$-Cycloalkyl-, Aryl- oder Aralkylgruppe darstellen.

5. Mittel, das in Form von Salben, Tinkturen, Crèmes, Pomaden, Pulvern, Tupfern, getränkten Tampons, Lösungen, Emulsionen, Lotionen, Gelen, Sprays oder wasserfreien oder wässrigen Suspensionen vorliegt, dadurch **gekennzeichnet,** daß es in einem zur topischen Anwendung geeigneten Träger mindestens eine Verbindung, erhältlich durch das Verfahren gemäß eines jeden der Ansprüche 1 bis 4, in Konzentrationen von 0,1 bis 10 Gew.%, bezogen auf Gesamtgewicht des Mittels, insbesondere von 0,2 bis 5 Gew.%, enthält.

6. Kosmetisches Mittel in Form von Lotionen, Gelen, Seifen, Shampoos,

dadurch **gekennzeichnet,** daß
es in einem physiologisch verträglichen Träger mindestens eine der Verbindungen, erhältlich durch das Verfahren gemäß eines jeden der Ansprüche 1 bis 4, in einer Konzentration von 0,01 bis 5 Gew.% enthält.

**7.** Mittel gemäß Anspruch 6,
dadurch **gekennzeichnet,** daß
es zusätzlich hydratisierende Mittel, antiseborrheische Mittel, Mittel zur Förderung des Haarwuchses, steroidische oder nicht-steroidische Anti-Entzündungsmittel, Carotinoide, Eicosantetrain-5,8,11,14- und Eicosantriin-5,8,11-Säuren sowie deren Ester und Amide enthält.

**8.** Mittel gemäß eines jeden der Ansprüche 5 bis 7,
dadurch **gekennzeichnet,** daß
es auch Konservierungs-, Stabilisierungs-, pH-Regulierungs-, Modifikationsmittel des osmotischen Drukkes, Emulgatoren, UV-A- und UV-B-Filter, Antioxidantien enthält.